# EUROPEAN PATENT APPLICATION

(11) **EP 1 878 730 A1**
(43) Date of publication of application: **16.01.2008**
(21) Application number: 06090122.0
(22) Date of filing: 12.07.2006
(51) Int. Cl.: C07D 261/04, C07D 413/12, A61K 31/42, A61P 35/00, A61P 17/06

(54) **Substituted isoxazolines, pharmaceutical compositions containing the same, methods of preparing the same, and uses of the same**

(71) Applicant: Bayer Schering Pharma Aktiengesellschaft, 13353 Berlin (DE)
(72) Inventor: Brittain, Dominic E.A., 10405 Berlin (DE); Bader, Benjamin, 13467 Berlin (DE); Lienau, Philip, 10115 Berlin (DE); Siemeister, Gerhard, 13503 Berlin (DE); Tatamiya, Takayuki, Mishinomiya, Hyogo, 662-0825 (JP); Weinmann, Hilmar, 16548 Glienicke/Nordbahn (DE); Huwe, Christoph, 13505 Berlin (DE)

(57) **Abstract**

The invention relates to substituted isoxazolines according to the general formula (I) : 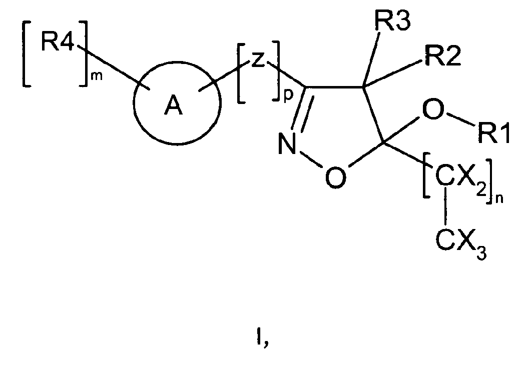 in which A, R1, R2, R3, R4, Z, X, m, n, and p, are given in the claims, and salts thereof, to pharmaceutical compositions comprising said substituted isoxazolines, to methods of preparing said substituted isoxazolines as well as the use thereof for manufacturing a pharmaceutical composition for the treatment of diseases known to be at least in part mediated by HDAC activity or whose symptoms are known to be alleviated by HDAC inhibitors.

## Description

The present invention relates to substituted isoxazoline compounds of general formula (I) and salts thereof, to pharmaceutical compositions comprising said substituted isoxazoline compounds, to methods of preparing said substituted isoxazolines, as well as to uses thereof.

Cancer forms a group of different diseases which are characterized by uncontrolled growth of a highly heterogeneous malignant cell population. The estimated worldwide incidence of different types of cancer is around 10 million, roughly half of which is in developed countries.¹ In spite of the large number of currently available chemotherapeutic drugs the medical need is still largely unmet. Therefore great efforts have been initiated for the identification of novel anticancer targets and the discovery of anticancer drugs.

The acetylation state of lysines of nucleosomal histones which modulates chromatin structure and regulates gene transcriptional activity is controlled by the antagonistic action of the two enzyme families histone deacetylases (HDACs) and histone acetyltransferases (HATs). Several structurally diverse HDAC inhibitors have now reached phase I and II clinical trials. Reviews on histone deacetylase as a new target for cancer chemotherapy ^{2,3,4,5} and the current status of HDAC inhibitors in clinical development have been published recently.^{6,7,8,9,10,11}

In humans there are three HDAC subclasses.¹² Class I enzymes, HDACs 1, 2, 3 and 8, are ranging in size from 42 - 55 kDa and share homology in their catalytic sites. Class II deacetylases (HDACs 4, 5, 6, 7, 9 and 10) are ranging in their molecular weights between 120 - 130 kDa and HDACs 4, 5, 7, 9 share homology in the C-terminal catalytic domain and a N-terminal regulatory domain.¹³ HDAC 6 which is closely related to HDAC 10 has two HDAC domains and has been demonstrated to deacetylate tubulin as a substrate.¹⁴ HDAC6 inhibition is also involved in the acetylation of other cellular targets like HSP90.¹⁵ HDAC 11 is another member of the HDAC enzymes which was recently cloned and characterized.¹⁶ It bears low similarity with class I or class II and therefore could not be classified in either class.

The SIR2 family of proteins is the third class of deacetylases. These enzmyes are dependent on NAD⁺ for activity whereas class I and II HDACs are using zinc-dependent mechanisms.

From the X-ray crystal structures of complexes of Trichostatin A and SAHA bound to HDAC-like protein (HDLP), a homologue of class I/II HDAC with ca. 35 % sequence identity, a structural rationale for HDAC inhibition has been derived.¹⁷ The hydroxamic acid moiety of Trichostatin A (TSA, **1**) occupies according to this structure a hydrophobic channel and binds a buried zinc ion. The first crystal structure of the human HDAC8 isoenzyme was published recently.¹⁸ Human HDAC8 structures complexed with four hydroxamate inhibitors have been determined. It was found that the inhibition of HDAC8 leads to the hyperacetylation of histones H3 and H4. From recent findings it was taken that HDAC8 may play a role in one of the most frequent types of acute myeloid leukemia (AML). This crystal structure sheds light on the catalytic mechanism of the HDACs, and on differences in substrate specificity across the HDAC family. A comparison of the structures of the four HDAC8-inhibitor complexes demonstrated considerable structural differences in the protein surface in the vicinity of the opening to the active site. These differences suggest that this region is highly flexible and able to structure changes to accommodate binding to a variety of different ligands. From a physiological point of view, this flexibility suggests that HDAC8 might be able to bind to acetylated lysines that are presented in a variety of structural contexts. Another independent crystal structure elucidation study of HDAC8 came to similar results.¹⁹ In addition this group demonstrated that knockdown of HDAC8 by RNA interference inhibits growth of human lung, colon, and cervical cancer cell lines, highlighting the importance of this HDAC subtype for tumor cell proliferation and therefore as a target for possible antitumor agents.

### 1. Hydroxamic acids

Hydroxamic acids constitute the largest class of HDAC inhibitors which is still rapidly growing. Reviews on solution- and solid-phase synthesis methods as well as potential therapeutic applications of hydroxamic acids have appeared recently. ^{20,21,22} Besides trichostatin A (TSA, H1) other first generation hydroxamic acid HDAC inhibitors like suberoyl anilide hydroxamic acid (SAHA), H2, Pyroxamide, H3, CBHA, H4, Oxamflatin, H5, and Scriptaid, H6, have been playing important roles as pharmacological tool compounds with some of them undergoing clinical trials.^{23,24} The most advanced of these compounds is SAHA which is currently in phase III clinical trials.²⁵

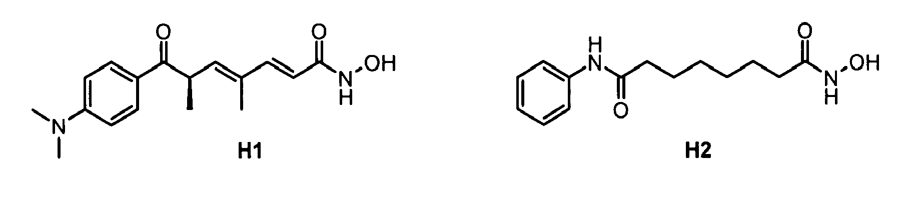

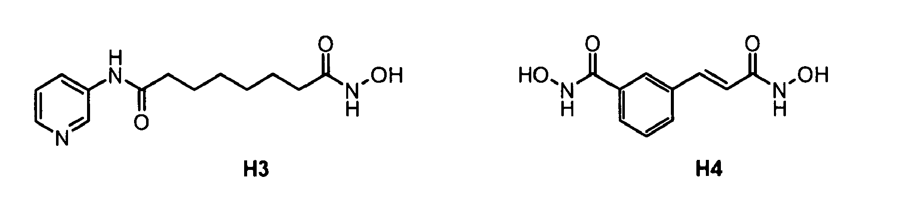

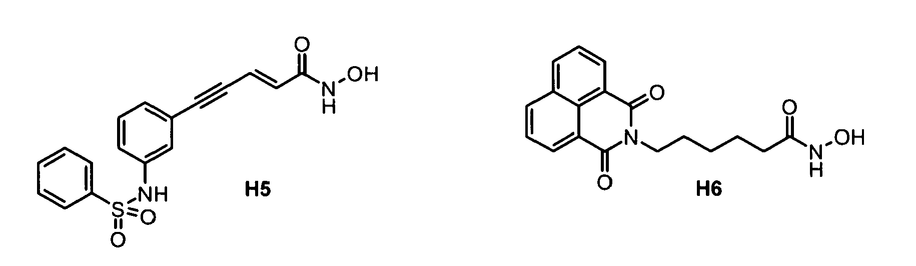

Further histone deacetylase inhibitors are reviewed in an article in Expert Opin. Ther. Patents, 2005, 15, 1677-1690, by Hilmar Weinmann and Eckhard Ottow, and is hereby incorporated in its entirety by reference.

HDAC inhibitors represent a prototype of molecularly targeted agents that perturb signal transduction, cell cycle-regulatory and survival-related pathways. Therefore it is not surprising that research activities surrounding HDAC inhibitors have been exponentially growing over the last couple of years and the field has developed into a highly competitive area. The by far biggest part of these research efforts is still dedicated to the compound classes of hydroxamic acids and o-phenylendiamine benzamides. Many highly potent compounds have been developed based on the early lead structures. Novel heterocyclic scaffolds like thiophenes, benzimidazoles and pyrimidines have been combined with components from existing HDAC inhibitors to increase potency and other pharmacological and physicochemical properties. Finally, newer generation HDAC inhibitors have been introduced into the clinics that are considerably more potent than their predecessors and are beginning to show early evidence of activity. Possible applications of HDAC inhibitors in psoriasis and neurodegenerative disorders,^{26,27} focussing especially on their anti-proliferative and anti-inflammatory activity reflect the growing recognition that HDAC inhibitors might be important therapeutic drugs in diseases other than cancer.

Despite the great progress made in the last few years two important challenges in the HDAC inhibitor research field are still not solved.

First of all the role of the various individual HDAC isoforms is still under investigation. It seems that each HDAC enzyme has a particular role in controlling transcription, the cell cycle, cell motility, DNA damage response, and senescence by deacetylating histone and non-histone proteins. Therefore enzyme sub-type specific inhibitors will be useful as tools for further studying the biology of these enzymes and eventually as new anticancer agents that control the specific function and the downstream pathway of HDACs with hopefully decreased toxicity. This is especially important as class II HDACs differ from class I HDACs depending on their tissue expression, subcellular localization, and biological roles. Class I HDACs are ubiquitously expressed, whereas class II enzymes display tissue-specific expression in humans and mice.²⁸ From the recently published experiments it can be taken that the focus is shifting more and more towards isoform-selective HDAC inhibitors and the first examples of such compounds which are at least selective for a couple of HDAC isoforms are just beginning to emerge. Further experimental studies of other effects of HDAC inhibitors which are lethal to cancer cells and may be unrelated to histone acetylation e.g. induction of oxidative damage by accumulation of reactive oxygen species (ROS) will also give rise to a more comprehensive picture of the various roles of this enzyme class.²⁹

Secondly, most of the current HDAC inhibitors still belong to the hydroxamic acid class. Although several hydroxamic acid derivatives have entered clinical studies there are still some discussions remaining whether these compounds will finally reach the market due to their pharmacokinetics, physicochemical and toxicological properties. Therefore the search for new and potent non-hydroxamic acid HDAC inhibitors as follow-up compounds is a still unsolved problem. However, it seems that the hydroxamic acid moiety combines in a relatively unique manner excellent zinc binding properties with the necessary hydrogen bond interactions to the amino acids at the catalytic site of the enzyme and therefore replacing hydroxamic acids so far had only limited success. Despite these challenges the ongoing search for novel potent and under physiological conditions stable substitutes for hydroxamic acids is both a scientifically and commercially rewarding task as it will open up competitive advantage in the exciting field of HDAC inhibitor research.

Surprisingly, it has been found unexpectedly by the inventors of the invention of the present patent application that the solution to the above-mentioned novel technical problem is achieved by providing compounds derived, in accordance with the present invention, from a class of substituted isoxazolines and salts thereof, methods of preparing substituted isoxazolines, a pharmaceutical composition containing said substituted isoxazolines, use of said substituted isoxazolines and a method for treating diseases with said substituted isoxazolines, all in accordance with the description, as defined in the claims of the present application.

In accordance with a main aspect, the present invention thus relates to compounds of general formula (I) : 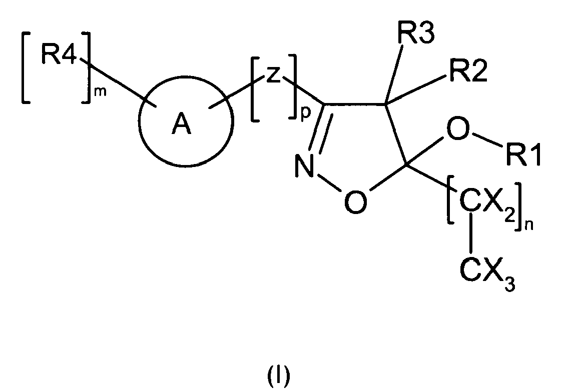 in which :
- R1: represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
- R2 and R3,: independently of each other, represent a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})2, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂, -C₁-C₆-alkyl-N(R^{a})₂, -C₁-C₆-alkyl-C(=O)R^{a}, -C₁-C₆-alkyl-C(=0)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a},-C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
or,
together, form a C₃-C₁₀-cycloalkyl or C₃-C₁₀-heterocycloalkyl ;
- A: represents an aryl or heteroaryl group ;
- X: represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
- Z: represents a C₂-C₆-alkenyl group, itself being optionally substituted, one or more times, in the same way or differently, with R5 ;
- R4: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=0)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})2, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- R5: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
- R^{a}: represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-atkylsulfonyl, C₁-C₆-alkylamino, C₂-C₆-alkenyl, C₂-C₆-alkynyl, C₃-C₁₀-cycloalkyl, C₃-C₁₀-heterocyctoalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
- R^{b}: represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyt, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂ ;
- R^{c}: represents, independently from each other, a substituent, which is identical or different, selected from the group comprising, preferably consisting of, a hydrogen, aryl and C₁-C₆-alkyl ;
- n: represents an integer of 0, 1, 2, 3, 4, 5, or 6 ;
- m: represents an integer of 0, 1, 2, 3, 4, or 5 ; and
- p: represents an integer of 0, 1, 2, 3, 4, or 5.

In accordance with a preferred embodiment, the present invention relates to compounds of formula I, *supra,* in which :
- R1: represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloatkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
- R2: represents a hydrogen atom ;
- R3: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyt, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- A: represents an aryl or heteroaryl group ;
- X: represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
- Z: represents a C₂-C₆-alkenyl group, itself being optionally substituted, one or more times, in the same way or differently, with R5 ;
- R4: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- R5: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkytsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
- R^{a}: represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
- R^{b}: represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂;
- R^{c}: represents, independently from each other, a substituent, which is identical or different, selected from the group comprising, preferably consisting of, a hydrogen, aryl and C₁-C₆-alkyl ;
- n: represents an integer of 0, 1, 2, 3, 4, 5, or 6 ;
- m: represents an integer of 0, 1, 2, 3, 4, or 5 ; and
- p: represents an integer of 0, 1, 2, 3, 4, or 5.

In accordance with a particularly preferred embodiment, the present invention relates to compounds of formula I, *supra,* in which :
- R1: represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyt, C₃-C₁₀-cycloalkyl, C₃-C₁₀-heterocyctoalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
- R2 and R3: represent a hydrogen atom ;
- A: represents an aryl or heteroaryl group ;
- X: represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
- Z: represents a C₂-C₆-alkenyl group, itself being optionally substituted, one or more times, in the same way or differently, with R5 ;
- R4: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-hatoalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- R5: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
- R^{a}: represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b}, C(=O)N(R^{b})₂, -C(=S)N(R^{b})2, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyt, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
- R^{b}: represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂;
- R^{c}: represents, independently from each other, a substituent, which is identical or different, selected from the group comprising, preferably consisting of, a hydrogen, aryl and C₁-C₆-alkyl ;
- n: represents an integer of 0, 1, 2, 3, 4, 5, or 6 ;
- m: represents an integer of 0, 1, 2, 3, 4, or 5 ; and
- p: represents an integer of 0, 1, 2, 3, 4, or 5.

In accordance with a more particularly preferred embodiment, the present invention relates to compounds of formula I, *supra,* in which :
- R1: represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
- R2 and R3: represent a hydrogen atom ;
- A: represents an aryl or heteroaryl group ;
- X: represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
- R4: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})2,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- R5: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})2, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
- R^{a}: represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyt, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
- R^{b}: represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂ ;
- R^{c}: represents, independently from each other, a substituent, which is identical or different, selected from the group comprising, preferably consisting of, a hydrogen, aryl and C₁-C₆-alkyl ;
- n: represents an integer of 0, 1, 2, 3, 4, 5, or 6 ;
- m: represents an integer of 0, 1, 2, 3, 4, or 5 ; and
- p: represents an integer of 0.

In accordance with a yet more particularly preferred embodiment, the present invention relates to compounds of formula I, *supra,* in which :
- R1: represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)_{N}(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
- R2 and R3: represent a hydrogen atom ;
- A: represents an aryl or heteroaryl group ;
- X: represents, independently of each other, a subsituent selected from the group comprising, preferably consisting of, a hydrogen atom, and a fluorine atom, wherein at least one X substituent is a fluorine atom ;
- R4: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
- R5: represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
- R^{a}: represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
- R^{b}: represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂ ;
- R^{c}: represents, independently from each other, a substituent, which is identical or different, selected from the group comprising, preferably consisting of, a hydrogen, aryl and C₁-C₆-alkyl ;
- n: represents an integer of 0, 1, 2, 3, 4, 5, or 6 ;
- m: represents an integer of 0, 1, 2, 3, 4, or 5 ; and
- p: represents an integer of 0.

The terms as mentioned herein below and in the claims have preferably the following meanings :

The term "alkyl" as used in the context of alkyl or alkylsulfonyl, alkylsulfinyl, alkylsulfenyl, alkylthio, alkylamino, for example, is to be understood as preferably meaning branched and unbranched alkyl, meaning *e.g.* methyl, ethyl, *n*-propyl, *iso*propyl, *n*-butyl, *iso*-butyl, *tert*-butyl, *sec*-butyl, pentyl, *iso*-pentyl, hexyl, heptyl, octyl, nonyl and decyl and the isomers thereof.

The term "haloalkyl" is to be understood as preferably meaning branched and unbranched alkyl, as defined *supra,* in which one or more of the hydrogen substituents is replaced in the same way or differently with halogen. Particularly preferably, said haloalkyl is, *e*.*g*. chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.

The term "alkoxy" is to be understood as preferably meaning branched and unbranched alkoxy, meaning e.g. methoxy, ethoxy, propyloxy, *iso*-propyloxy, butyloxy, *iso*-butyloxy, *tert*-butyloxy, *sec*-butyloxy, pentyloxy, *iso*-pentyloxy, hexyloxy, heptyloxy, octyloxy, nonyloxy, decyloxy, undecyloxy and dodecyloxy and the isomers thereof.

The term "haloalkoxy" is to be understood as preferably meaning branched and unbranched alkoxy, as defined *supra,* in which one or more of the hydrogen substituents is replaced in the same way or differently with halogen, e.g. chloromethoxy, fluoromethoxy, pentafluoroethoxy, fluoropropyloxy, difluoromethyloxy, trichloromethoxy, 2,2,2-trifluoroethoxy, bromobutyloxy, trifluoromethoxy, iodoethoxy, and isomers thereof.

The term "cycloalkyl" is to be understood as preferably meaning a C₃-C₁₀ cycloalkyl group, more particularly a saturated cycloalkyl group of the indicated ring size, meaning *e.g.* a cyclopropyl, cyclobutyl, cyclopentyl, cyclohexyl, cycloheptyl, cyclooctyl, cyclononyl, or cyclodecyl group ; and also as meaning an unsaturated cycloalkyl group containing one or more double bonds in the C-backbone, e.g. a C₃-C₁₀ cycloalkenyl group, such as, for example, a cyclopropenyl, cyclobutenyl, cyclopentenyl, cyclohexenyl, cycloheptenyl, cyclooctenyl, cyclononenyl, or cyclodecenyl group, wherein the linkage of said cyclolaklyl group to the rest of the molecule can be provided to the double or single bond.

The term "heterocycloalkyl" is to be understood as preferably meaning a C₃-C₁₀ cycloalkyl group, as defined *supra*, featuring the indicated number of ring atoms, wherein one or more ring atoms are heteroatoms such as NH, NR, oxygen or sulphur, or carbonyl groups, or, -otherwise stated - in a Cₙ-cycloalkyl group one or more carbon atoms are replaced by these heteroatoms to give such Cₙ cycloheteroalkyl group, and in addition in each case can be benzocondensed. Thus such group refers e.g. to a three-membered heterocycloalkyl, expressed as -C₃-heterocycloalkyl such as oxyranyl. Other examples of heterocycloalkyls are oxetanyl (C₄), aziridinyl (C₃), azetidinyl (C₄), tetrahydrofuranyl (C₅), [1,3]dioxolanyl (C₅), pyrrolidinyl (C₅), morpholinyl (C₆), dithianyl (C₆), thiomorpholinyl (C₆), piperazinyl (C₆), trithianyl (C₆) and chinuclidinyl (C₈).

The term "halogen" or "Hal" is to be understood as preferably meaning fluorine, chlorine, bromine, or iodine.

The term "alkenyl" is to be understood as preferably meaning branched and unbranched alkenyl, e.g. a vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl, or 2-methyl-prop-1-en-1-yl group.

The term "alkynyl" is to be understood as preferably meaning branched and unbranched alkynyl, e.g. an ethynyl, prop-1-yn-1-yl, but-1-yn-1-yl, but-2-yn-1-yl,or but-3-yn-1-yl group.

The term "aryl" as used in the context of aryl or aryloxy, for example, is defined in each case as having 3-12 carbon atoms, preferably 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred. It is further understood that in the case in which said aryl group is substituted with one or more substituents, said substituent(s) may occupy any one or more positions on said aryl ring(s). Particularly, in the case of aryl being a phenyl group, said substituent(s) may occupy one or both ortho positions, one or both meta positions, or the para position, or any combination of these positions.

The term "heteroaryl" as used in the context of heteroaryl or heteroaryloxy, for example, is understood as meaning an aromatic ring system which comprises 3-16 ring atoms, preferably 5 or 6 or 9 or 10 atoms, and which contains at least one heteroatom which may be identical or different, said heteroatom being such as oxygen, nitrogen or sulphur, and can be monocyclic, bicyclic, or tricyclic, and in addition in each case can be benzocondensed. Preferably, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl etc., and benzo derivatives thereof, such as, e.g., benzofuranyl, benzothienyl, benzoxazolyl, benzimidazolyl, benzotriazolyl, indazolyl, indolyl, isoindolyl, etc.; or pyridyl, pyridazinyl, pyrimidinyl, pyrazinyl, triazinyl, etc., and benzo derivatives thereof, such as, for example, quinolinyl, isoquinolinyl, etc.; or azocinyl, indolizinyl, purinyl, etc., and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc. It is further understood that in the case in which said heteroaryl group is substituted with one or more substituents, said substituent(s) may occupy any one or more positions on said heteroaryl ring(s). Particularly, in the case of heteroaryl being a pyridyl group, for example, said substituent(s) may occupy any one or more of positions 2, 3, 4, 5, and/or 6 with respect to the nitrogen atom in the pyridine ring.

The term "alkylene", as used herein in the context of the compounds of general formula (I) is to be understood as meaning an optionally substituted alkyl chain or "tether", having 1, 2, 3, 4, 5, or 6 carbon atoms, i.e. an optionally substituted - CH₂- ("methylene" or "single membered tether" or e.g. -C(Me)₂-), -CH₂-CH₂-("ethylene", "dimethylene", or "two-membered tether"), -CH₂-CH₂-CH₂-("propylene", "trimethylene", or "three-membered tether"), -CH₂-CH₂-CH₂-CH₂-("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂- ("hexylene", "hexamethylene", or six-membered tether") group. Preferably, said alkylene tether is 1, 2, 3, 4, or 5 carbon atoms, more preferably 1 or 2 carbon atoms.

The term "cycloalkylene", as used herein in the context of the compounds of general formula (I) is to be understood as meaning an optionally substituted cycloalkyl ring, having 3, 4, 5, 6, 7, 8, 9 or 10, preferably 3, 4, 5, or 6, carbon atoms, *i.e.* an optionally substituted cyclopropyl, cyclobutyl, cyclopenyl, cyclohexyl, cycloheptyl, cyclooctyl, cyclononyl, or cyclodecyl ring, preferably a cyclopropyl, cyclobutyl, cyclopentyl or cyclohexyl ring.

The term "heterocycloalkylene", as used herein in the context of the compounds of general formula (I) is to be understood as meaning a cycloalkylene ring, as defined *supra,* but which contains at least one heteroatom which may be identical or different, said heteroatom being such as oxygen, nitrogen or sulphur.

The term "arylene", as used herein in the context of the compounds of general formula (I) which include the groups D and E, is to be understood as meaning an optionally substituted monocyclic or polycyclic arylene aromatic system e.g. arylene, naphthylene and biarylene, preferably an optionally substituted phenyl ring or "tether", having 6 or 10 carbon atoms. More preferably, said arylene tether is a ring having 6 carbon atoms. If the term "arylene" is used it is to be understood that the linking residues can be arranged to each other in ortho-, para- and meta-position, e.g.an optionally substituted moiety of structure : 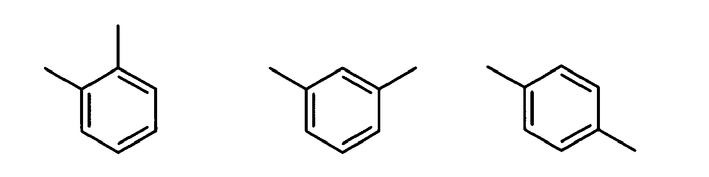 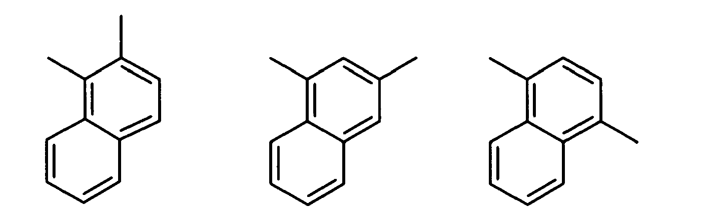 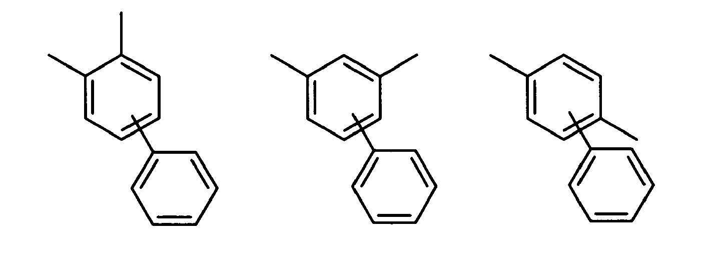 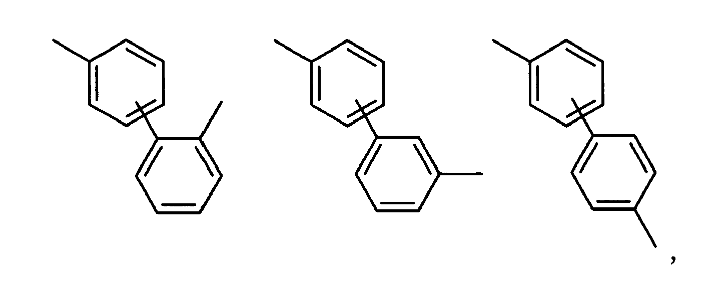 in which linking positions on the rings are shown as non-attached bonds.

The term "heteroarylene", as used herein in the context of the compounds of general formula (I) which include the groups D and E, is to be understood as meaning an optionally substituted monocyclic or polycyclic heteroarylene aromatic system, e.g. heteroarylene, benzoheteroarylene, preferably an optionally substituted 5-membered heterocycle, such as, for example, furan, pyrrole, thiazole, oxazole, isoxazole, or thiophene or "tether", or a 6-membered heterocycle, such as, for example, pyridine, pyrimidine, pyrazine, pyridazine. More preferably, said heteroarylene tether is a ring having 6 carbon atoms, e.g. an optionally substituted structure as shown *supra* for the arylene moieties, but which contains at least one heteroatom which may be identical or different, said heteroatom being such as oxygen, nitrogen or sulphur. If the term "heteroarylene" is used it is to be understood that the linking residues can be arranged to each other in ortho-, para- and meta-position.

As used herein, the term "C₁-C₆", as used throughout this text, e.g. in the context of the definition of "C₁-C₆-alkyl", or "C₁-C₆-alkoxy", is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, e.g. C₁-C₆, C₂-C₅ , C₃-C₄ , C₁-C₂, C₁-C₃ , C₁-C₄, C₁-C₅ C₁-C₆; preferably C₁-C₂, C₁-C₃, C₁-C₄, C₁-C₅, C₁-C_{6;} more preferably C₁-C₄.

Similarly, as used herein, the term "C₂-C₆", as used throughout this text, e.g. in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, e.g. C₂-C₆, C₃-C₅, C₃-C₄, C₂-C₃, C₂-C₄, C₂-C_{5;} preferably C₂-C₃.

As used herein, the term "C₃-C₁₀", as used throughout this text, e.g. in the context of the definitions of "C₃-C₁₀-cycloalkyl" or "C₃-C₁₀-heterocycloalkyl", is to be understood as meaning a cycloalkyl group having a finite number of carbon atoms of 3 to 10, i.e. 3, 4, 5, 6, 7, 8, 9 or 10 carbon atoms, preferably 3, 4, 5 or 6 carbon atoms. It is to be understood further that said term "C₃-C₁₀" is to be interpreted as any sub-range comprised therein, *e.g.* C₃-C₁₀, C₄-C₉, C₅-C₈ , C₆-C₇ ; preferably C₃-C₆.

As used herein, the term "C₃-C₆", as used throughout this text, e.g. in the context of the definitions of "C₃-C₆-cycloalkyl" or "C₃-C₆-heterocycloalkyl", is to be understood as meaning a cycloalkyl group having a finite number of carbon atoms of 3 to 6, i.e. 3, 4, 5, or 6 carbon atoms. It is to be understood further that said term "C₃-C₆" is to be interpreted as any sub-range comprised therein, e.g. C₃-C₄, C₄-C₆ , C₅-C₆ .

As used herein, the term "C₆-C₁₁", as used throughout this text, e.g. in the context of the definitions of "C₆-C₁₁-aryl", is to be understood as meaning an aryl group having a finite number of carbon atoms of 5 to 11, *i.e.* 5, 6, 7, 8, 9, 10 or 11 carbon atoms, preferably 5, 6, or 10 carbon atoms. It is to be understood further that said term "C₆-C₁₁" is to be interpreted as any sub-range comprised therein, e.g. C₅-C₁₀, C₆-C₉, C₇-C₈; Preferably C₅-C₆.

As used herein, the term "C₅-C₁₀", as used throughout this text, e.g. in the context of the definitions of "C₅-C₁₀-heteroaryl", is to be understood as meaning a heteroaryl group having a finite number of carbon atoms of 5 to 10, in addition to the one or more heteroatoms present in the ring i.e. 5, 6, 7, 8, 9, or 10 carbon atoms, preferably 5, 6, or 10 carbon atoms. It is to be understood further that said term "C₅-C₁₀" is to be interpreted as any sub-range comprised therein, e.g. C₆-C₉, C₇-C₈, C₇-C₈; preferably C₅-C₆.

As used herein, the term "C₁-C₃", as used throughout this text, *e.g.* in the context of the definitions of "C₁-C₃-alkylene", is to be understood as meaning an alkylene group as defined *supra* having a finite number of carbon atoms of 1 to 3, *i.e.* 1, 2, or 3. It is to be understood further that said term "C₁-C₃" is to be interpreted as any sub-range comprised therein, e.g. C₁-C₂, or C₂-C₃.

The term "one or more", used in the context of the present invention, e.g. in the phrase "substituted one or more times", is understood as meaning one, two, three, or four times.

The term "leaving group" as used in the context of the method aspects of the present invention, is to be understood as meaning a group which is displaced from a compound in a substitution or an elimination reaction, for example a halogen atom, a trifluoromethanesulphonate ("triflate") group, alkoxy, methanesulphonate, p-toluenesulphonate, etc..

The term "isomers" is to be understood as meaning chemical compounds with the same number and types of atoms as another chemical species. There are two main classes of isomers, constitutional isomers and stereoisomers.

The term "constitutional isomers" is to be understood as meaning chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major subclasses of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers which still have a different constitution, are another sub-class of diastereomers, the best known of which are simple *cis - trans* isomers. Clearly, when it is possible for a compound of the present invention to exist in such isomeric forms, the present invention covers single isomers, and any mixture, e.g. racemic mixtures, of such isomers, whether they be isolated or not.

In order to limit different types of isomers from each other reference is made to IUPAC Rules Section E *(Pure Appl Chem* 45, 11-30, 1976).

The compound according to Fomula (I) can exist in free form or in a salt form. A suitably pharmaceutically acceptable salt of the isoxazolines of the present invention may be, for example, an acid-addition salt of an isoxazoline of the invention which is sufficiently basic, for example, an acid-addition salt with, for example, an inorganic or organic acid, for example hydrochloric, hydrobromic, sulphuric, phosphoric, trifluoroacetic, para-toluenesulphonic, methylsulphonic, citric, tartaric, succinic or maleic acid. In addition, another suitably pharmaceutically acceptable salt of an isoxazoline of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethylglucamine, ethyl-glucamine, lysine, 1,6-hexadiamine, ethanolamine, glucosamine, sarcosine, serinol, tris-hydroxy-methyl-aminomethane, aminopropandiol, sovak-base, 1-amino-2,3,4-butantriol.

The compound according to Formula (I) can exist as N-oxides which are defined in that at least one nitrogen of the compounds of the general Formula (I) may be oxidized.

The compound according to Formula (I) can exist as solvates, in particular as hydrate, wherein the compound according to Formula (I) may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, are possible hemi-, (semi-), mono-, sesqui-, di-, tri-, tetra-, penta- etc. solvates or hydrates, respectively.

As used herein, the term *"in vivo* hydrolysable ester" is understood as meaning an in *vivo* hydrolysable ester of a compound of formula (I) containing a carboxy or hydroxyl group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, e.g. methoxymethyl, C₁-C₆ alkanoyloxymethyl esters, e.g. pivaloyloxymethyl, phthalidyl esters, C₃-C₈ cycloalkoxy-carbonyloxy-C₁-C₆ alkyl esters, e.g. 1-cyclohexylcarbonyloxyethyl ; 1,3-dioxolen-2-onylmethyl esters, e.g. 5-methyl-1,3-dioxolen-2-onylmethyl ; and C₁-C₆-alkoxycarbonyloxyethyl esters, e.g. 1-methoxycarbonyloxyethyl, and may be formed at any carboxy group in the compounds of this invention. An in *vivo* hydrolysable ester of a compound of formula (I) containing a hydroxyl group includes inorganic esters such as phosphate esters and [alpha]-acyloxyalkyl ethers and related compounds which as a result of the in *vivo* hydrolysis of the ester breakdown to give the parent hydroxyl group. Examples of [alpha]-acyloxyalkyl ethers include acetoxymethoxy and 2,2-dimethylpropionyloxymethoxy. A selection of in *vivo* hydrolysable ester forming groups for hydroxyl include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

Further, another embodiment of the present invention relates to the use of the intermediate compounds, for example compounds of formulae A and B, as mentioned *supra,* for the preparation of a compound of general formula (I) as defined *supra.*

The compounds of the present invention can be used in treating diseases known to be at least in part mediated by HDAC activity or whose symptoms are known to be alleviated by HDAC inhibitors (*vide supra* for specific examples).

Therefore, another aspect of the present invention is a use of the compound of general formula (I) described *supra* for manufacturing a pharmaceutical composition for the treatment of diseases known to be at least in part mediated by HDAC activity or whose symptoms are known to be alleviated by HDAC inhibitors.

Preferably, the use is in the treatment of diseases caused by increased cell proliferation. These include, but are not limited to, cancer, psoriasis, fibroproliferative disorders (e.g. liver fibrosis), smooth muscle cell proliferation disorders (e.g. arteriosclerosis, restenosis).

Another preferred use is in the treatment of diseases, wherein the diseases are inflammatory diseases and conditions treatable by immune modulation (e.g. rheumatoid arthritis, autoimmune diabetes, lupus, allergies).

A further use is in the treatment of diseases caused by expanded polyglutamine repeats resulting in histone hypoacetylation including, but not limited to, neurodegenerative disorders (e.g. Huntington's disease).

Another use is in the treatment of diseases involving angiogenesis, wherein the diseases are, but not limited to, cancer, psoriasis, rheumatoid arthritis, retinal diseases such as diabetic retinopathy, age-related macular degeneration, interstitial keratitis and rubeotic glaucoma.

A further use is in the treatment of fungal and parasitic infections including, but not limited to, malaria, toxoplasmosis, coccidiosis and protozoal infections.

Another use is in the treatment of diseases involving haematopoietic disorders including, but not limited to, anaemia, sickle cell anaemia and thalassemia.

Yet another aspect of the invention is a method of treating a disease known to be at least in part mediated by HDAC activity or whose symptoms are known to be alleviated by HDAC inhibitors, by administering an effective amount of a compound of general formula (I) described *supra.*

Preferably, the use is in the treatment of diseases caused by increased cell proliferation. These include, but are not limited to, cancer, psoriasis, fibroproliferative disorders (e.g. liver fibrosis), smooth muscle cell proliferation disorders (e.g. arteriosclerosis, restenosis).

Another preferred use is in the treatment of diseases, wherein the diseases are inflammatory diseases and conditions treatable by immune modulation (e.g. rheumatoid arthritis, autoimmune diabetes, lupus, allergies).

A further use is in the treatment of diseases caused by expanded polyglutamine repeats resulting in histone hypoacetylation including, but not limited to, neurodegenerative disorders (e.g. Huntington's disease).

Another use is in the treatment of diseases involving angiogenesis, wherein the diseases are, but not limited to, cancer, psoriasis, rheumatoid arthritis, retinal diseases such as diabetic retinopathy, age-related macular degeneration, interstitial keratitis and rubeotic glaucoma.

A further use is in the treatment of fungal and parasitic infections including, but not limited to, malaria, toxoplasmosis, coccidiosis and protozoal infections.

Another use is in the treatment of diseases involving haematopoietic disorders including, but not limited to, anaemia, sickle cell anaemia and thalassemia.

The compounds of the present invention can thus be applied for the treatment of diseases caused by increased cell proliferation. These include, but are not limited to, inflammatory diseases, cardiomyocyte hypertrophy, primary and metastatic cancers of different origin (including those triggered by viral infections such as EBV, HIV, hepatitis B and C and KSHV), fibrosis of the liver, lung, kidney, heart and skin caused by myofibroblast proliferation and increased production of extracellular matrix proteins.

The compounds of the present invention can be used in particular in therapy and prevention of tumour growth and metastases, especially in solid tumours of all indications and stages with or without pre-treatment. However, it is not restricted to tumour therapy but is also of great value for the treatment of other diseases known to be at least in part mediated by HDAC activity or whose symptoms are known to be alleviated by HDAC inhibitors. These include, but are not limited to, cancer, psoriasis, fibroproliferative disorders (e.g. liver fibrosis), smooth muscle cell proliferation disorders (e.g. arteriosclerosis, restenosis). It also includes, but is not limited to, inflammatory diseases and conditions treatable by immune modulation (e.g. rheumatoid arthritis, autoimmune diabetes, lupus, allergies). A further use is in the treatment of diseases caused by expanded polyglutamine repeats resulting in histone hypoacetylation including, but not limited to, neurodegenerative disorders (e.g. Huntington's disease). Another use is in the treatment of diseases involving angiogenesis, wherein the diseases are, but not limited to, cancer, psoriasis, rheumatoid arthritis, retinal diseases such as diabetic retinopathy, age-related macular degeneration, interstitial keratitis and rubeotic glaucoma. Additional use is in the treatment of fungal and parasitic infections including, but not limited to, malaria, toxoplasmosis, coccidiosis and protozoal infections. Another use is in the treatment of diseases involving haematopoietic disorders including, but not limited to, anaemia, sickle cell anaemia and thalassemia. It is therapeutically valuable for diseases, whose treatment involves control over transcription, the cell cycle, cell motility, DNA damage response or senescence.

Another aspect of the present invention is a pharmaceutical composition which contains a compound of Formula (I) or pharmaceutically acceptable salts thereof, N-oxides, solvates, hydrates, isomers or mixtures of isomers thereof, in admixture with one or more suitable excipients. This composition is particularly suited for the treatment of diseases known to be at least in part mediated by HDAC activity or whose symptoms are known to be alleviated by HDAC inhibitors as explained above.

In order that the compounds of the present invention be used as pharmaceutical products, the compounds or mixtures thereof may be provided in a pharmaceutical composition, which, as well as the compounds of the present invention for enteral, oral or parenteral application contain suitably pharmaceutically acceptable organic or inorganic inert base material, e.g. purified water, gelatin, gum Arabic, lactate, starch, magnesium stearate, talcum, vegetable oils, polyalkylenglycol, etc.

The pharmaceutical compositions of the present invention may be provided in a solid form, e.g. as tablets, dragées, suppositories, capsules or in liquid form, e.g. as a solution, suspension or emulsion. The pharmaceutical composition may additionally contain auxiliary substances, e.g. preservatives, stabilisers, wetting agents or emulsifiers, salts for adjusting the osmotic pressure or buffers.

For parenteral applications, (including intravenous, subcutaneous, intramuscular, intravascular or infusion), sterile injection solutions or suspensions are preferred, especially aqueous solutions of the compounds in polyhydroxyethoxy containing castor oil.

The pharmaceutical compositions of the present invention may further contain surface active agents, e.g. salts of gallenic acid, phosphorlipids of animal or vegetable origin, mixtures thereof and liposomes and parts thereof.

For oral application tablets, dragées or capsules with talcum and/or hydrocarbon-containing carriers and binders, e.g. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 to 1,500 mg. A dose can be administered as unit dose or in part thereof and distributed over the day. Accordingly the optimum dosage may be determined by the practitioner who is treating any particular patient.

It is possible for compounds of general formula(I)of the present invention to be used alone or, indeed in combination with one or more further drugs, particularly anti-cancer drugs or compositions thereof. Particularly, it is possible for said combination to be a single pharmaceutical composition entity, e.g. a single pharmaceutical formulation containing one or more compounds according to general formula(I)together with one or more further drugs, particularly anti-cancer drugs, or in a form, e.g. a "kit of parts", which comprises, for example, a first distinct part which contains one or more compounds according to general formula I, and one or more further distinct parts each containing one or more further drugs, particularly anti-cancer drugs. More particularly, said first distinct part may be used concomitantly with said one or more further distinct parts, or sequentially. The additional drug(s) may or may not be HDAC inhibitors.

Another aspect of the present invention is a method which may be used for preparing the compounds according to the present invention.

The following Table lists the abbreviations used in this paragraph and in the Examples section as far as they are not explained within the text body. NMR peak forms are stated as they appear in the spectra, possible higher order effects have not been considered.

Chemical names were generated using AutoNom2000 as implemented in MDL ISIS Draw.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash column chromatography, using for example prepacked silica gel cartridges, e.g. from Separtis such as Isolute® Flash silica gel or Isolute® Flash NH₂ silica gel in combination with a Flashmaster II autopurifier (Argonaut/Biotage) and eluants such as gradients of hexane/EtOAc or DCM/ethanol. In some cases, the compounds may be purified by preparative HPLC using for example a Waters autopurifier equipped with a diode array detector and/or on-line electrospray ionization mass spectrometer in combination with a suitable prepacked reverse phase column and eluants such as gradients of water and acetonitrile which may contain additives such as trifluoroacetic acid or aqueous ammonia.

| Abbreviation | Meaning |
|---|---|
| Ac | acetyl |
| AIBN | α,α'-azobisisobutyronitrile |
| Boc | *tert*-butyloxycarbonyl |
| br | broad |
| c- | cyclo- |
| Cl | chemical ionisation |
| d | Doublet |
| dd | doublet of doublet |
| dt | double triplet |
| | |
| DCM | dichloromethane |
| DIPEA | N,N-diisopropylethyl amine |
| DMAP | N, N-dimethylaminopyridine |
| DMF | N,N-dimethylformamide |
| DMSO | dimethyl sulphoxide |
| EDCI | 1-(3-dimethylaminopropyl)-3-ethylcarbodiimide hydrochloride |
| eq. | equivalent |
| ESI | electrospray ionisation |
| GP | general procedure |
| h | hours |
| HPLC | high performance liquid chromatography |
| LC-MS | liquid chromatography mass spectrometry |
| m | multiplet |
| mc | centred multiplet |
| MS | mass spectrometry |
| NMR | nuclear magnetic resonance spectroscopy : chemical shifts (δ) are given in ppm. |
| OTf | trifluoromethylsulphonyl- |
| Pg | protecting group |
| ppm | parts per million |
| q | quartet |
| rf | at reflux |
| r.t. or rt | room temperature |
| s | singlet |
| sept. | septet |
| t | triplet |
| T3P | 1-propanephosphonic acid cyclic anhydride |
| TEA | triethylamine |
| TFA | trifluoroacetic acid |
| THF | tetrahydrofuran |

The following General Synthetic Routes shown in the following Schemes and the following General Experimental Section giving general procedures illustrate general synthetic routes to the compounds of general formula I of the invention and are not intended to be limiting. Specific examples are described in the subsequent paragraph.

### General Synthetic Routes:

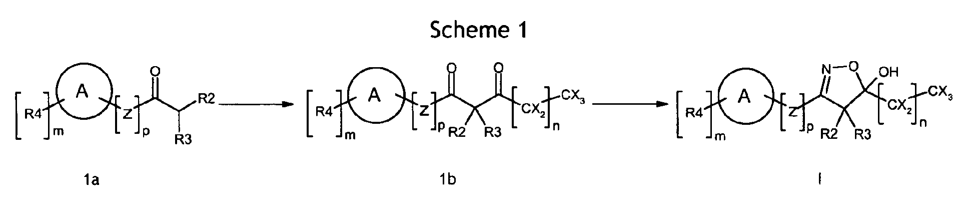

Ketones of formula 1a may be reacted with a suitable base, preferably in a solvent, for example sodium in ethanol, followed by the addition of a fluoro-substituted acetyl electrophile, for example trifluoroacetic acid ethyl ester, to give intermediate substituted 1,3-diones of formula 1b. These, in turn, may be condensed with hydroxylamine to yield the substituted isoxazolines of formula I, for example by following literature procedures (J. Org. Chem., 1995, 60, 3907-3909). 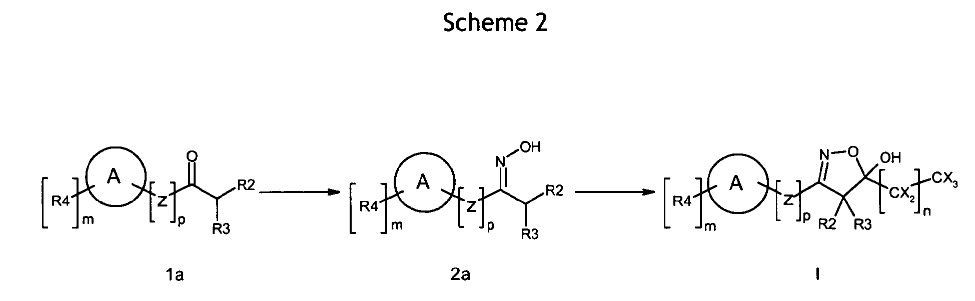 Ketones of formula 1a may be condensed with hydroxylamine to generate oximes of formula 2a. These, in turn, may be reacted with a suitable base, preferably in a solvent, for example n-butyllithium in THF, followed by the addition of a fluoro-substituted acetyl electrophile, for example trifluoroacetic acid ethyl ester, to give substituted isoxazolines of formula I, for example by following literature procedures *(*Bioorg. Med. Chem. Lett., 2005, 15, 5562-5566). 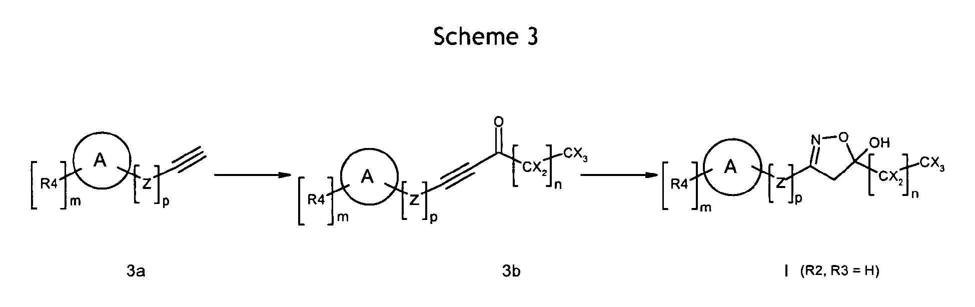 Monosubstituted acetylenes of formula 3a may be reacted with a suitable base, preferably in a solvent, for example n-butyllithium in THF, followed by the addition of a fluoro-substituted acetyl electrophile, for example trifluoroacetic acid ethyl ester, to give intermediate substituted alkynones of formula 3b. These, in turn, may be condensed with hydroxylamine to yield the substituted isoxazolines of formula I wherein R2, R3 = H, for example by following literature procedures *(*Tetrahedron Lett., 1989, 30, 16, 2049-2052). 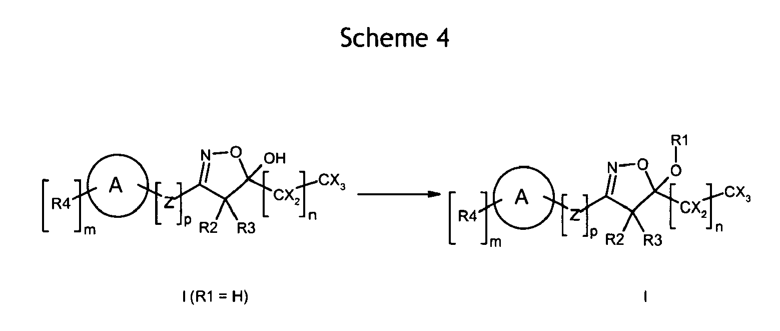 The above-generated substituted isoxazolines of formula I wherein R1 = H may then be reacted with a suitable base, for example pyridine, followed by the addition of an electrophile, for example acetic anhydride, to give further substituted isoxazolines of formula I.

### SYNTHESIS OF KEY INTERMEDIATES

In the subsequent paragraphs detailed procedures for the synthesis of key intermediates for compounds of the present invention are described.

### General Experimental:

1-(4-Bromomethyl-phenyl)-ethanone 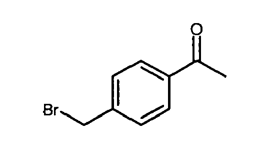 A solution of 1-p-tolyl-ethanone (16 ml, 120 mmol) in 1-Butyl-3-methyl-3H-imidazol-1-ium hexafluorophosphate (20 ml) was degassed with a stream of Argon and sonication for 8 minutes. N-Bromosuccinimide (25.6 g, 144mmol) and AIBN (0.97 g, 5.91 mmol) were added to the reaction mixture, which was similarly degassed for a further 8 minutes and then stirred at 60°C for 2½ h. The reaction mixture was cooled, extracted with diethyl ether, and the organic phase washed with brine, dried (Na₂SO₄), filtered and concentrated *in vacuo* to give crude 1-(4-Bromomethyl-phenyl)-ethanone (27.3 g) which was used without further purification.
¹H-NMR (CDCl₃): δ = 2.58 (m, 3H); 4.51 (s, 2H); 7.48 (d, 2H); 7.94 (d, 2H) ppm.

1-(3-Bromomethyl-phenyl)-ethanone 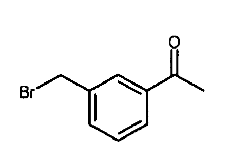 By proceeding in a similar manner to the previous procedure but using 1-m-Tolyl-ethanone, crude 1-(3-Bromomethyl-phenyl)-ethanone was prepared.
¹H-NMR (CDCl₃): δ = 2.53 (m, 3H); 4.53 (s, 2H); 7.47 (m, 1 H); 7.60 (dt, 1 H); 7.88 (dt, 1H); 7.97 (s, 1 H) ppm.

1-(4-Azidomethyl-phenyl)-ethanone 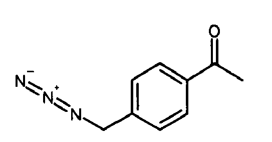 Sodium azide (8.43 g, 130 mmol) was added to a solution of crude 1-(4-Bromomethyl-phenyl)-ethanone in DMF (187 ml) at 0°C. After 3h at 0°C, the reaction mixture was warmed to room temperature and stirred for a further 2h, before being diluted with water and extracted with ethyl acetate. The organic phase was separated, washed with brine, dried (Na₂SO₄), filtered and concentrated *in vacuo* and the residue purified by flash column chromatography (hexane/ethyl acetate) to give 1-(4-Azidomethyl-phenyl)-ethanone (14.7 g, 70% over two steps). ¹H-NMR (CDCl₃): δ = 2.62 (s, 3H); 4.42 (s, 2H); 7.41 (d, 2H); 7.97 (d, 2H) ppm.

1-(3-Azidomethyl-phenyl)-ethanone 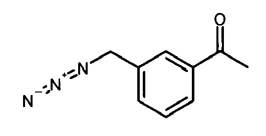 By proceeding in a similar manner to the previous procedure but using 1-(3-Bromomethyl-phenyl)-ethanone, 1-(3-Azidomethyl-phenyl)-ethanone (16.44 g, 78% yield over two steps) was prepared.
¹H-NMR (CDCl₃): δ = 2.65 (s, 3H); 4.43 (s, 2H); 7.46-7.57 (m, 2H); 7.89-7.96 (m, 2H) ppm.

1-(4-Azidomethyl-phenyl)-4,4,4-trifluoro-butane-1,3-dione 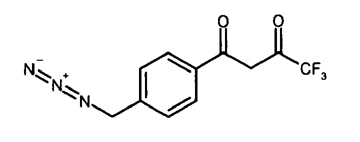 A solution of 1-(4-Azidomethyl-phenyl)-ethanone (10.5 g, 60.0 mmol) in ethanol (18 ml) was added slowly to a suspension of sodium (3.22 g, 140 mmol) in ethanol (110 ml), followed by dropwise addition of trifluoro-acetic acid ethyl ester (10.8 ml, 90.1 mmol). After stirring for 2h, the reaction mixture was quenched with a 1 N solution of aqueous hydrochloric acid and extracted with ethyl acetate. The organic phase was washed with water then brine, dried (Na₂SO₄), filtered and concentrated *in vacuo* to give crude 1-(4-Azidomethyl-phenyl)-4,4,4-trifluorobutane-1,3-dione (18.4 g) which was used without further purification.
¹H-NMR (CDCl₃): δ = 4.47 (s, 2H); 6.59 (s, 1 H); 7.47 (d, 2H); 7.97 (d, 2H) ppm.

1-(3-Azidomethyl-phenyl)-4,4,4-trifluoro-butane-1,3-dione 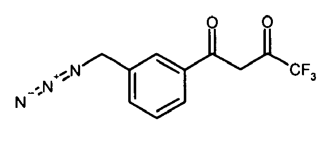 By proceeding in a similar manner to the previous procedure but using 1-(3-Azidomethyl-phenyl)-ethanone, crude 1-(3-Azidomethyl-phenyl)-4,4,4-trifluorobutane-1, 3-dione was prepared.
¹H-NMR (CDCl₃): δ = 4.48 (s, 2H); 6.59 (s, 1H); 7.52-7.64 (m, 2H); 7.88-7.95 (m, 2H) ppm.

1-(4-Benzyloxy-phenyl)-4,4,4-trifluoro-butane-1,3-dione 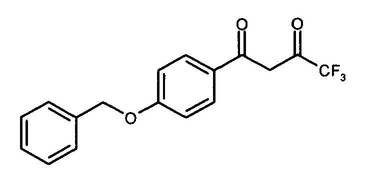 By proceeding in a similar manner to the previous procedure but using 1-(4-Benzyloxy-phenyl)-ethanone, crude 1-(4-Benzyloxy-phenyl)-4,4,4-trifluoro-butane-1,3-dione was prepared.
¹H-NMR (CDCl₃): δ = 5.27 (s, 2H); 6.53 (s, 1H); 7.08 (d, 2H); 7.34-7.47 (m, 5H); 7.95 (d, 2H) ppm.

3-(4-Azidomethyl-phenyl)-5-trifluoromethyl-4,5-dihydro-isoxazol-5-ol 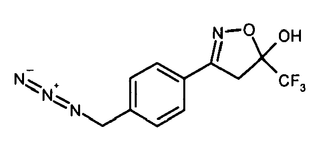 A solution of hydroxylamine hydrochloride (4.34 g, 62.5 mmol) in water (51 ml) and a 2N solution of aqueous sodium hydroxide (33 ml, 67 mmol) was slowly added to a solution of crude 1-(4-Azidomethyl-phenyl)-4,4,4-trifluoro-butane-1,3-dione in ethanol (150 ml). After stirring for 3h at 60°C, the reaction mixture was quenched with an aqueous solution of ammonium chloride and extracted with ethyl acetate. The organic phase was separated, washed with brine, dried (Na₂SO₄), filtered and concentrated *in vacuo* to give crude 3-(4-Azidomethyl-phenyl)-5-trifluoromethyl-4,5-dihydro-isoxazol-5-ol (18.2 g) which was used without further purification.
¹H-NMR (CDCl₃): δ = 3.52 (d, 1H); 3.74 (d, 1H); 4.41 (s, 2H); 7.42 (d, 2H); 7.70 (d, 2H) ppm.

3-(3-Azidomethyl-phenyl)-5-trifluoromethyl-4,5-dihydro-isoxazol-5-ol 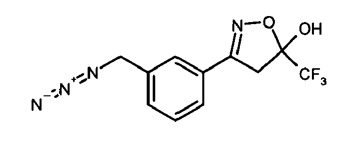 By proceeding in a similar manner to the previous procedure but using 1-(3-Azidomethyl-phenyl)-4,4,4-trifluoro-butane-1,3-dione, crude 3-(3-Azidomethyl-phenyl)-5-trifluoromethyl-4,5-dihydro-isoxazol-5-ol was prepared.
¹H-NMR (CDCl₃): δ = 3.55 (d, 1H); 3.75 (d, 1H); 4.42 (s, 2H); 7.43-7.50 (m, 2H); 7.60-7.67 (m, 2H) ppm.

3-(4-Benzyloxy-phenyl)-5-trifluoromethyl-4,5-dihydro-isoxazol-5-ol 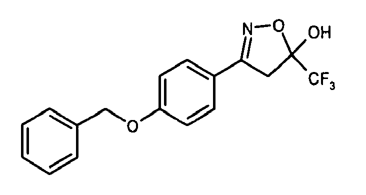 By proceeding in a similar manner to the previous procedure but using 1-(3-Azidomethyl-phenyl)-4,4,4-trifluoro-butane-1,3-dione and working-up by addition of water, filtering off the precipitate and drying, 3-(4-Benzyloxy-phenyl)-5-trifluoromethyl-4,5-dihydro-isoxazol-5-ol was prepared.
¹H-NMR (CDCl₃): δ = 3.50 (d, 1 H); 3.72 (d, 1 H); 5.13 (s, 2H); 7.04 (d, 2H); 7.32-7.47 (m, 5H); 7.63 (d, 2H) ppm.

3-(4-Aminomethyl-phenyl)-5-trifluoromethyl-4,5-dihydro-isoxazol-5-ol 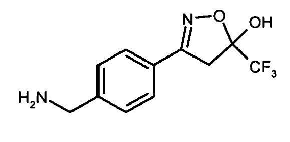 Triphenylphosphine (19.5g, 74.4 mmol) was added to a solution of crude 3-(4-Azidomethyl-phenyl)-5-trifluoromethyl-4,5-dihydro-isoxazol-5-ol in THF (93ml) and water (65 ml) and vigorously stirred. After 4 hours, the reaction mixture was concentrated in vacuo and purified by flash column chromatography.(MeOH/DCM 10-30%) to give 3-(4-Aminomethyl-phenyl)-5-trifluoromethyl-4,5-dihydro-isoxazol-5-ol (8.11 g, 49% over 3 steps).
¹H-NMR (DMSO-d6): δ = 3.49 (d, 1H); 3.74 (s, 2H); 3.87 (d, 1H); 7.41 (d, 2H); 7.63 (d, 2H) ppm.

3-(3-Aminomethyl-phenyl)-5-trifluoromethyl-4,5-dihydro-isoxazol-5-ol 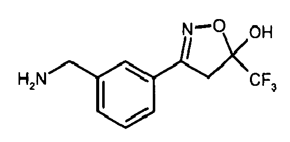 By proceeding in a similar manner to the previous procedure but using 3-(3-Azidomethyl-phenyl)-5-trifluoromethyl-4, 5-dihydro-isoxazol-5-ol, 3-(3-Aminomethyl-phenyl)-5-trifluoromethyl-4,5-dihydro-isoxazol-5-ol (6.5 g, 41% yield over 3 steps) was prepared.
¹H-NMR (DMSO-d6): δ = 3.52 (d, 1H); 3.71 (s, 2H); 3.87 (d, 1H); 7.34-7.39 (m, 1H); 7.42-7.45 (m, 1 H); 7.53 (m, 1 H); 7.67 (s, 1 H) ppm.

The present invention thus provides a method of preparing a compound of general formula (I) *supra,* said method comprising the step of allowing an intermediate amine compound of general formula A : 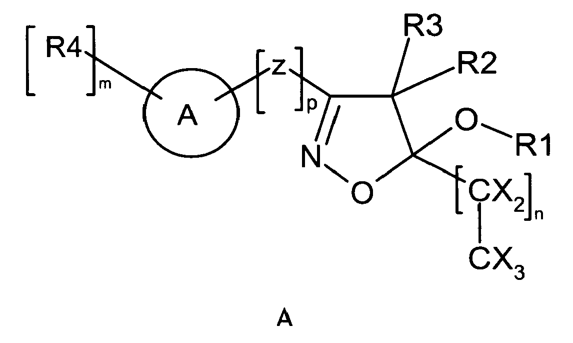 in which R4 is -CH₂NH₂ and m is 1, the remaining definitions being as given *supra,*
to react with a compound of formula B : 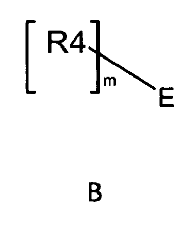 wherein
- R4: represents a substituent selected from the group comprising, preferably consisting of sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a},-C(=O)N(R^{a})₂ -C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a},-C(=O)OR^{a}, -C(=O)N(R^{a})₂, -C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5, wherein R^{a} and R5 are defined *supra ;*
to provide a compound of general formula I : 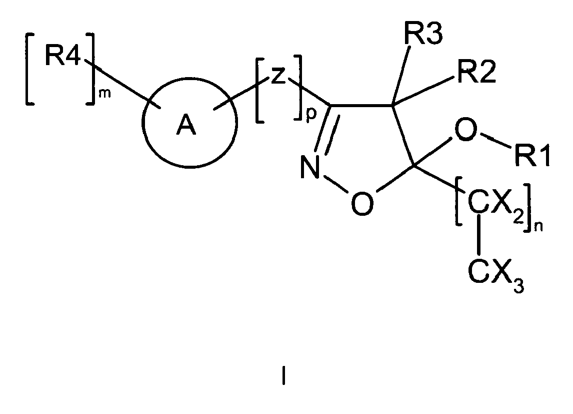 in which formula I, the definitions of A, R1, R2, R3, R4, X, Z, m, n, and p, are given for the main aspect, *supra,* and in which formulae A and B, the definitions of A, R1, R2, R3, X, Z, m, n, and p, are given for each case *supra,* and in which formula B, E is a leaving group. For example, specifically, without the invention being limited thereto :

### 1. Amides, sulfonamides, ureas, thioureas

To the amine of formula A (0.15 mmol) in dimethylformamide (0.4 mL) the compound of formula B (0.165 mmol) in dimethylformamide (0.3 mL) and 4-dimethylaminopyridine (0.16 mmol) in dimethylformamide (0.2 mL) were sucessively added, and the mixture was stirred at 100 °C bath temperature for 12 h. For work-up, the mixture was cooled to room temperature, diluted with methanol (1 mL) and concentrated. The residue was purified using preparative HPLC-MS and the products were characterized using analytical HPLC-MS (column LiChroCart Purospher Star 125-4 (125x4.5 mm, RP18e, 5 µm); gradient 5-95% acetonitrile (0.1% trifluoroacetic acid) in water (0.1% trifluoroacetic acid) (10 min.); flow rate 1.2 mL/min; MS ES+).

In accordance with this method, the following compounds were prepared :

| **Example** | **Structure** | **MW** | **Mot. peak** | **RT (min)** |
|---|---|---|---|---|
| 1 | 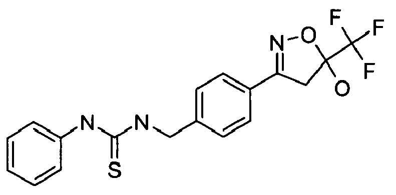 | 395.41 | 396.03 | 8.81 |
| 2 | 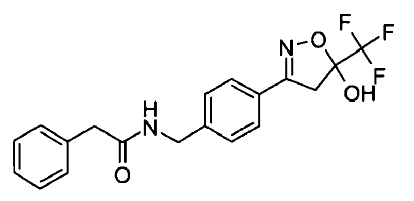 | 378.35 | 379.04 | 8.36 |
| 3 | 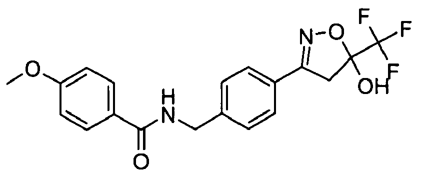 | 394.35 | 395.02 | 8.38 |
| 4 | 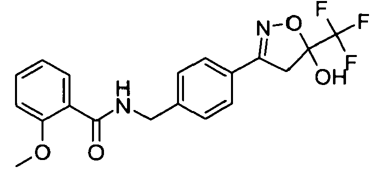 | 394.35 | 395.02 | 8.60 |
| 5 | 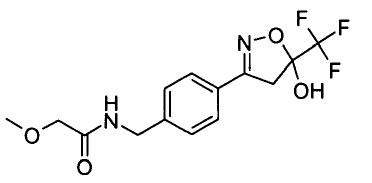 | 332.28 | 333.07 | 7.18 |
| 6 | 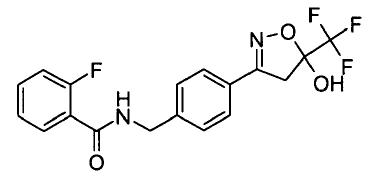 | 382.32 | 383.01 | 8.52 |
| 7 | 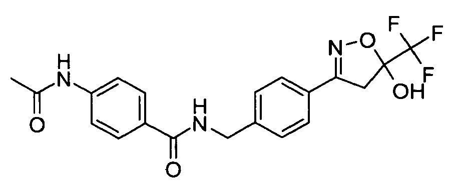 | 421.38 | 422.01 | 7.36 |
| 8 | 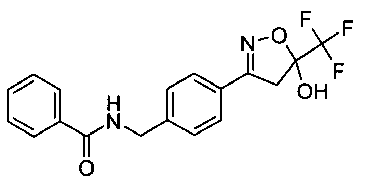 | 364.33 | 365.03 | 8.30 |
| 9 | 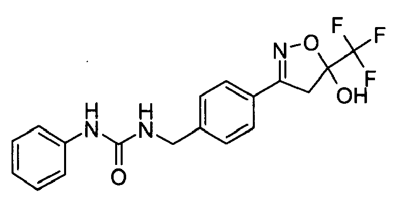 | 379.34 | 380.04 | 8.43 |
| 10 | 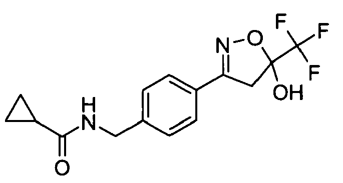 | 328.29 | 329.05 | 7.55 |
| 11 | 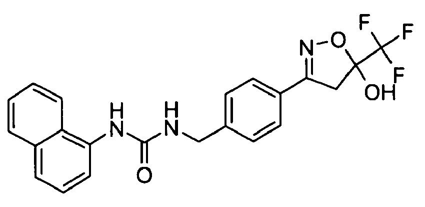 | 429.40 | 430.01 | 8.85 |
| 12 | 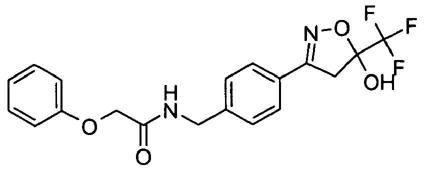 | 394.35 | 395.02 | 8.72 |
| 13 | 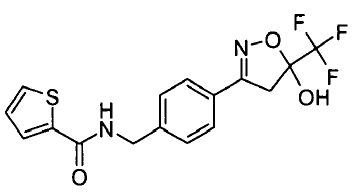 | 370.35 | 370.98 | 8.27 |
| 14 | 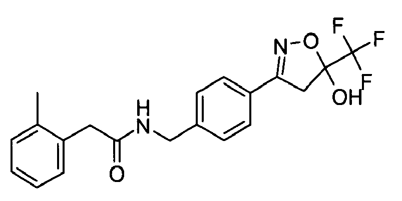 | 392.38 | 393.03 | 8.69 |
| 15 | 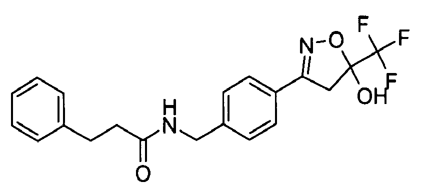 | 392.38 | 393.05 | 8.53 |
| 16 | 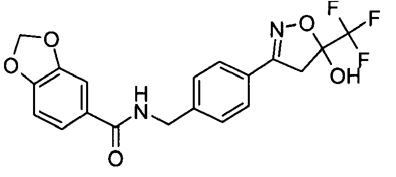 | 408.34 | 408.99 | 8.35 |
| 17 | 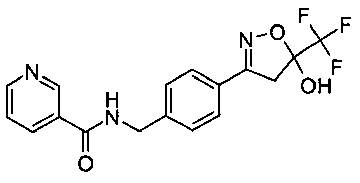 | 365.31 | 366.02 | 6.53 |
| 18 | 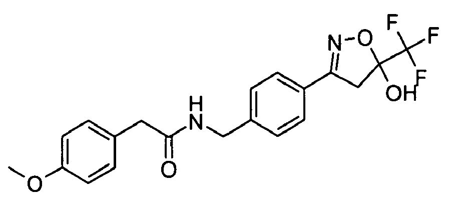 | 408.38 | 409.04 | 8.38 |
| 19 | 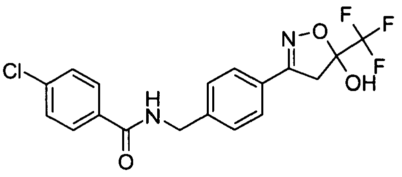 | 398.77 | 398.96 | 9.01 |
| 20 | 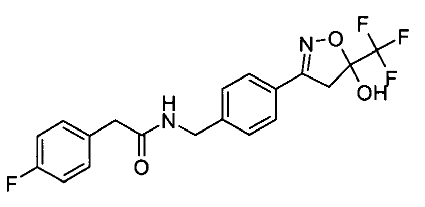 | 396.34 | 397.04 | 8.43 |
| 21 | 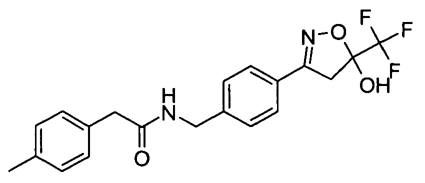 | 392.38 | 393.06 | 8.78 |
| 22 | 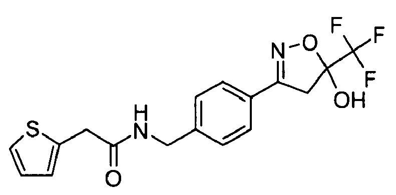 | 384.38 | 384.99 | 8.27 |
| 23 | 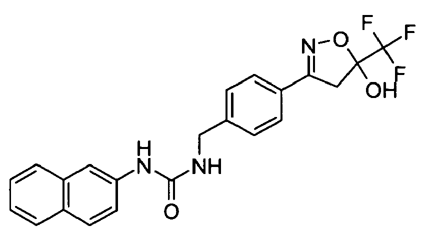 | 429.40 | 430.01 | 9.18 |
| 24 | 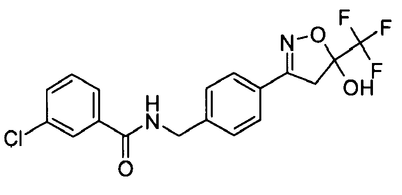 | 398.77 | 398.99 | 9.06 |
| 25 | 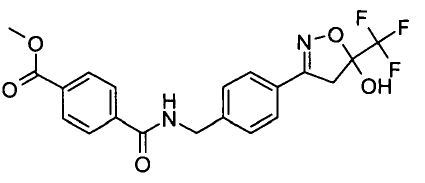 | 422.36 | 423.01 | 8.49 |
| 26 | 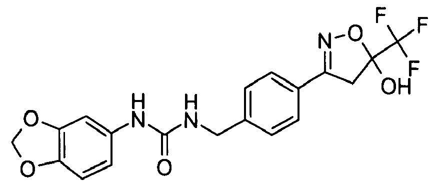 | 423.35 | 423.97 | 8.20 |
| 27 | 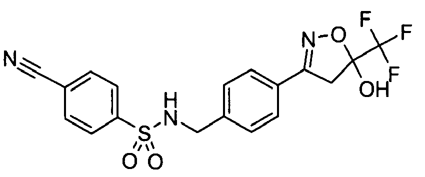 | 425.39 | 425.93 | 8.66 |
| 28 | 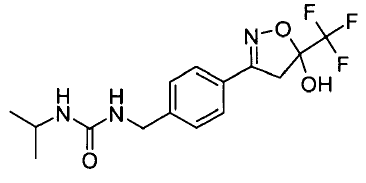 | 345.32 | 346.09 | 7.51 |
| 29 | 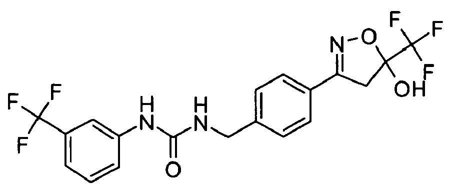 | 447.34 | 447.99 | 9.28 |
| 30 | 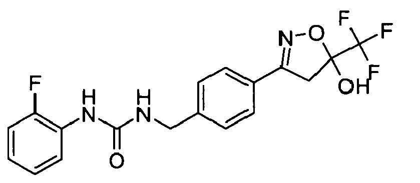 | 397.33 | 397.99 | 8.68 |
| 31 | 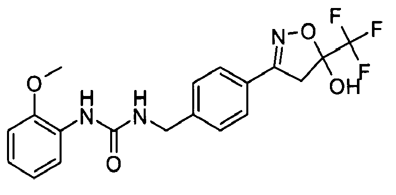 | 409.37 | 410.00 | 8.68 |
| 32 | 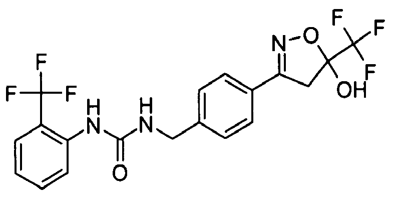 | 447.34 | 447.95 | 8.99 |
| 33 | 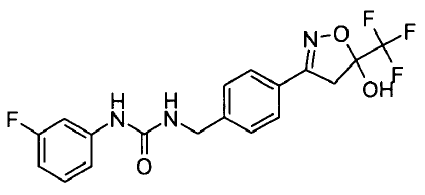 | 397.33 | 398.02 | 8.66 |
| 34 | 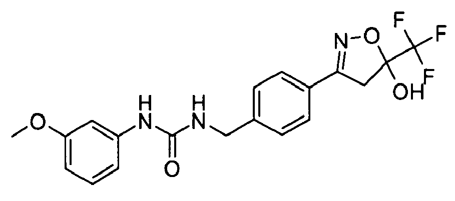 | 409.37 | 410.01 | 8.50 |
| 35 | 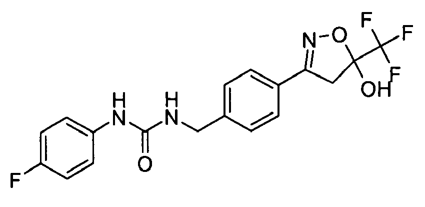 | 397.33 | 397.99 | 8.55 |
| 36 | 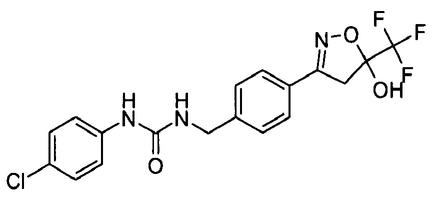 | 413.79 | 413.96 | 9.05 |
| 37 | 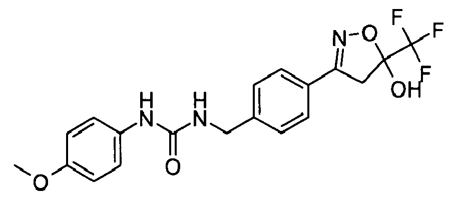 | 409.37 | 410.02 | 8.18 |
| 38 | 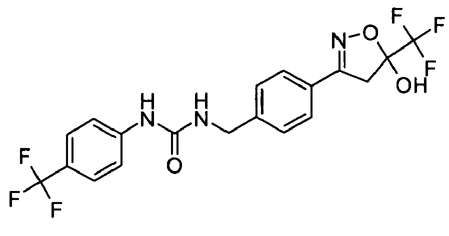 | 447.34 | 447.98 | 9.40 |
| 39 | 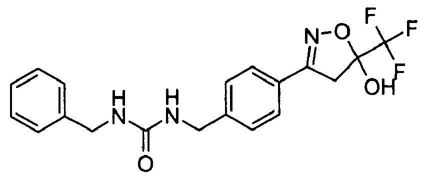 | 393.37 | 394.04 | 8.23 |
| 40 | 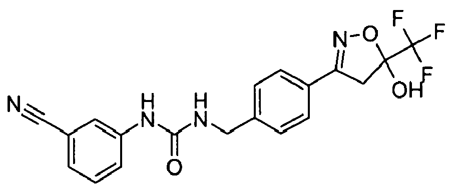 | 404.35 | 405.01 | 8.46 |
| 41 | 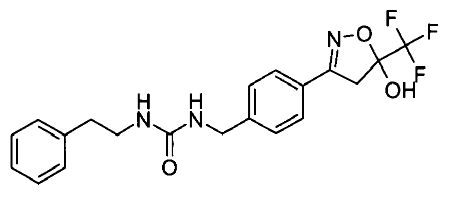 | 407.40 | 408.05 | 8.40 |
| 42 | 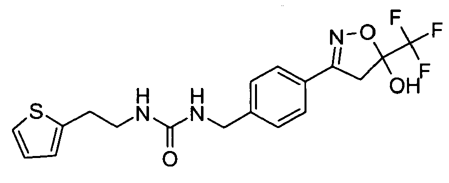 | 413.42 | 413.98 | 8.33 |
| 43 | 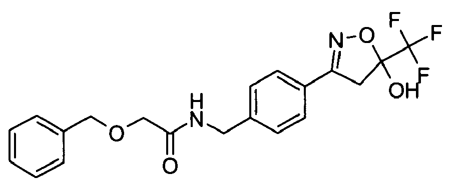 | 408.38 | 409.02 | 8.73 |
| 44 | 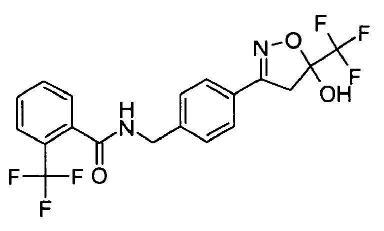 | 432.33 | 432.96 | 8.68 |
| 45 | 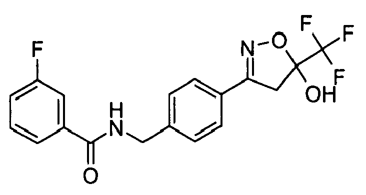 | 382.32 | 383.01 | 8.66 |
| 46 | 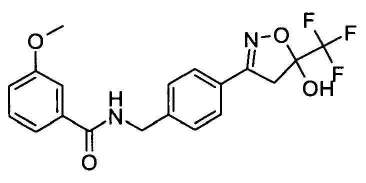 | 394.35 | 395.02 | 8.52 |
| 47 | 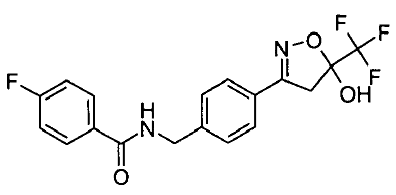 | 382.32 | 383.01 | 8.50 |
| 48 | 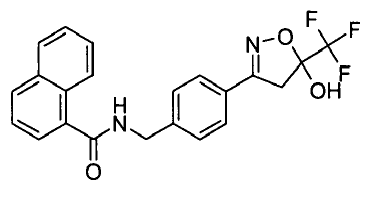 | 414.39 | 415.03 | 9.06 |
| 49 | 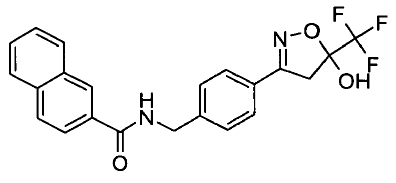 | 414.39 | 415.02 | 9.25 |
| 50 | 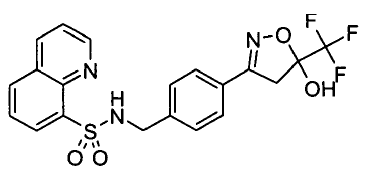 | 451.43 | 451.97 | 8.70 |
| 51 | 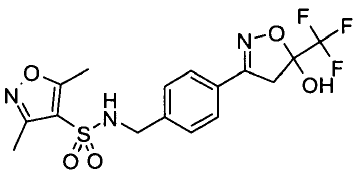 | 419.38 | 419.96 | 8.48 |
| 52 | 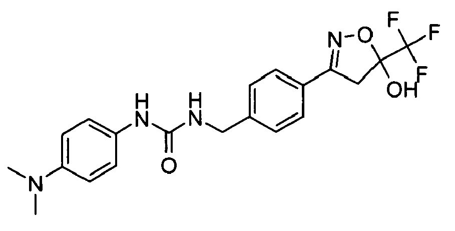 | 422.41 | 423.03 | 5.90 |
| 53 | 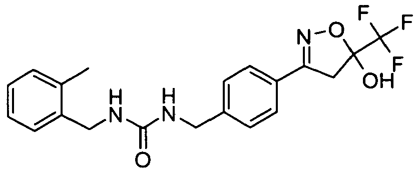 | 407.40 | 408.04 | 8.55 |
| 54 | 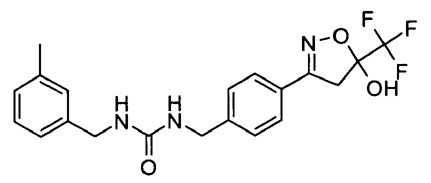 | 407.40 | 408.05 | 8.56 |
| 55 | 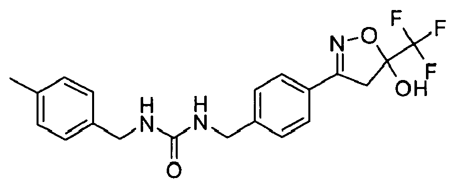 | 407.40 | 408.05 | 8.53 |
| 56 | 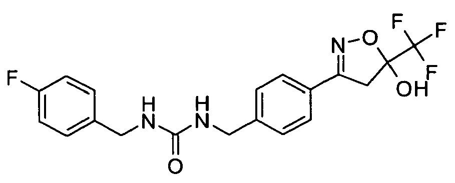 | 411.36 | 412.01 | 8.38 |
| 57 | 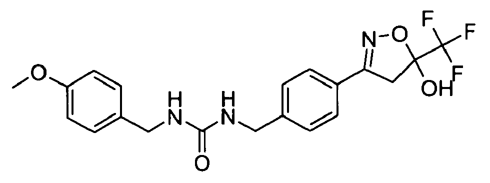 | 423.40 | 424.03 | 8.20 |
| 58 | 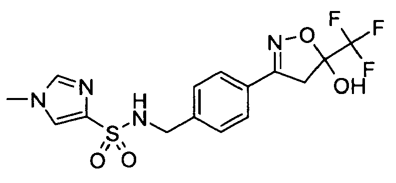 | 404.37 | 404.96 | 7.13 |
| 59 | 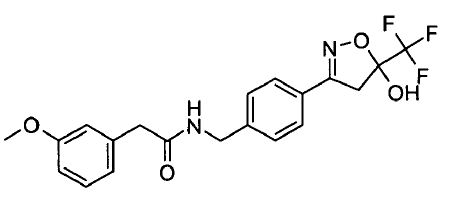 | 408.38 | 409.04 | 8.33 |
| 60 | 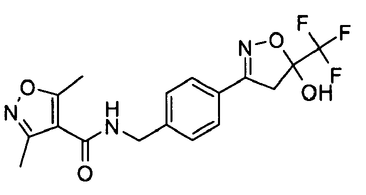 | 383.33 | 384.01 | 7.91 |
| 61 | 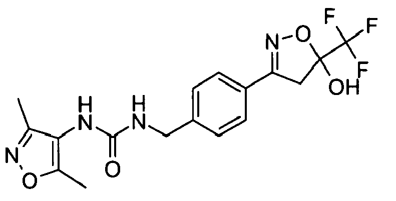 | 398.34 | 399.02 | 7.30 |
| 62 | 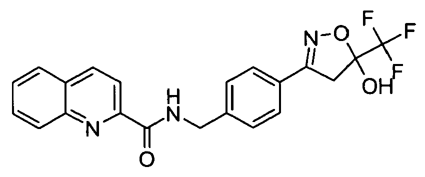 | 415.38 | 416.01 | 9.40 |
| 63 | 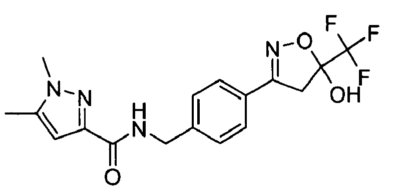 | 382.35 | 383.03 | 7.70 |
| 64 | 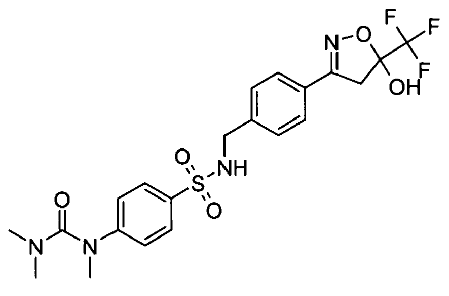 | 500.50 | 501.00 | 8.05 |
| 65 | 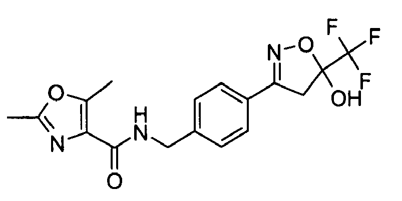 | 383.33 | 384.01 | 8.28 |
| 66 | 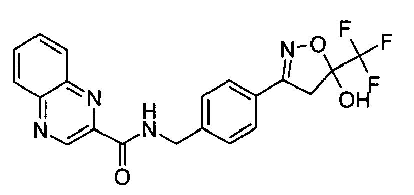 | 416.36 | 416.99 | 8.68 |
| 67 | 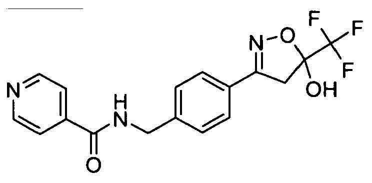 | 365.31 | 366.02 | 6.40 |
| 68 | 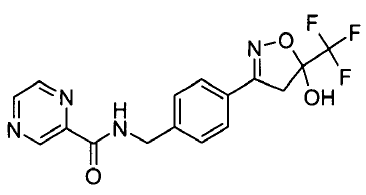 | 366.30 | 367.03 | 7.51 |
| 69 | 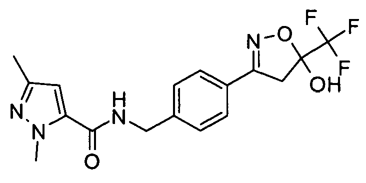 | 382.35 | 383.04 | 7.78 |
| 70 | 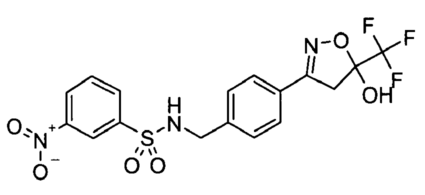 | 445.38 | 445.94 | 8.78 |
| 71 | 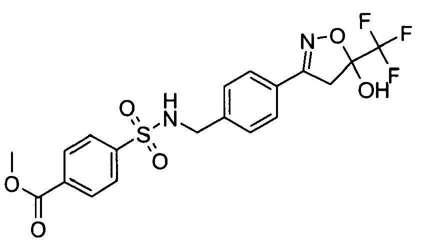 | 458.42 | 458.92 | 8.86 |
| 72 | 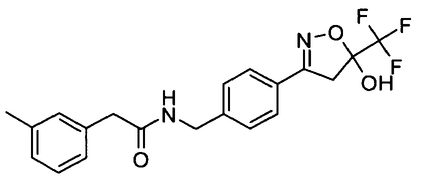 | 392.38 | 393.05 | 8.75 |
| 73 | 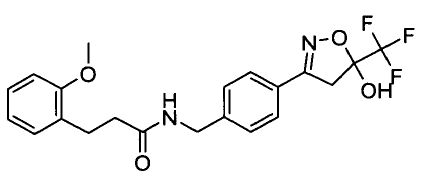 | 422.41 | 423.02 | 8.74 |
| 74 | 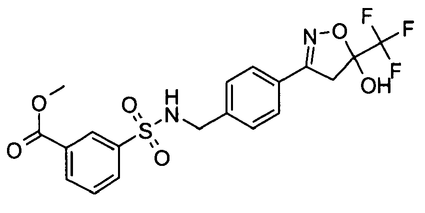 | 458.42 | 458.95 | 8.78 |
| 75 | 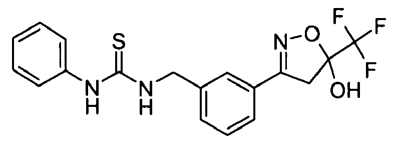 | 395.41 | 395.99 | 8.86 |
| 76 | 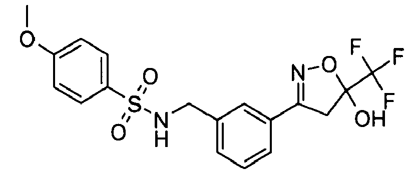 | 430.41 | 430.99 | 8.78 |
| 77 | 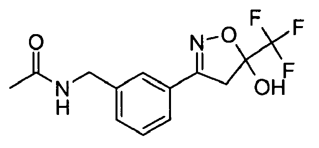 | 302.26 | 303.10 | 6.84 |
| 78 | 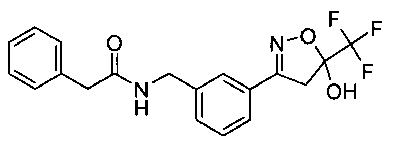 | 378.35 | 379.04 | 8.45 |
| 79 | 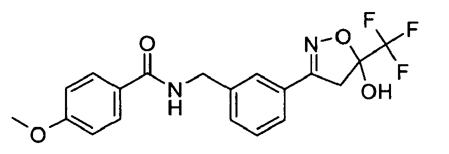 | 394.35 | 395.02 | 8.47 |
| 80 | 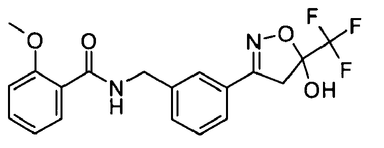 | 394.35 | 395.02 | 8.71 |
| 81 | 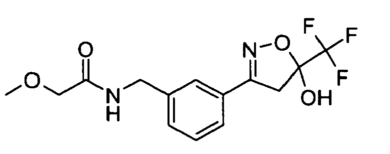 | 332.28 | 333.07 | 7.23 |
| 82 | 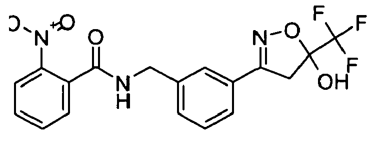 | 409.32 | 409.99 | 8.25 |
| 83 | 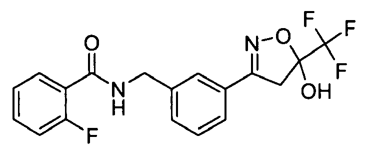 | 382.32 | 383.01 | 8.55 |
| 84 | 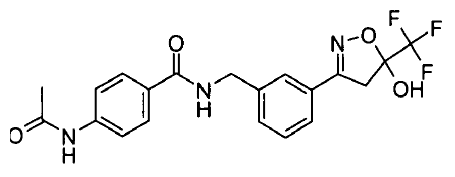 | 421.38 | 422.01 | 7.35 |
| 85 | 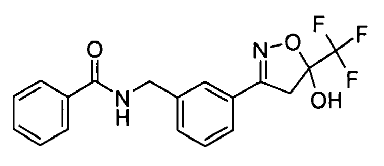 | 364.33 | 365.02 | 8.43 |
| 86 | 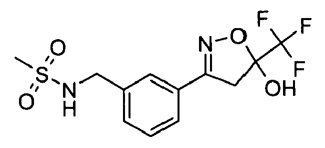 | 338.31 | 338.99 | 7.43 |
| 87 | 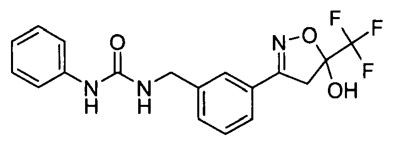 | 379.34 | 380.00 | 8.46 |
| 88 | 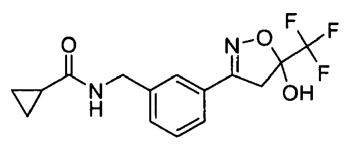 | 328.29 | 329.05 | 7.57 |
| 89 | 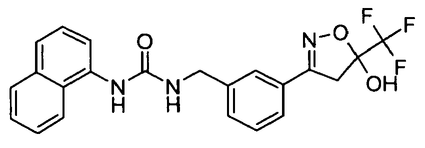 | 429.40 | 429.99 | 8.97 |
| 90 | 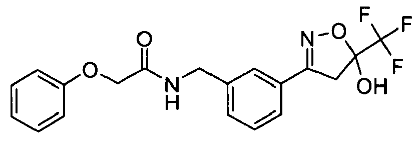 | 394.35 | 395.01 | 8.72 |
| 91 | 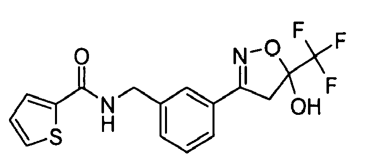 | 370.35 | 370.96 | 8.33 |
| 92 | 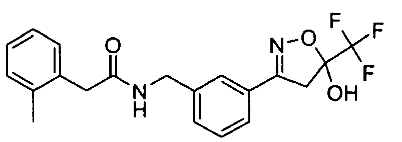 | 392.38 | 393.01 | 8.70 |
| 93 | 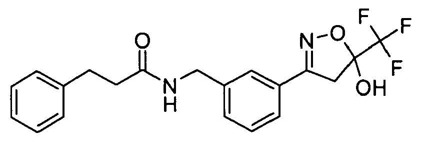 | 392.38 | 393.01 | 8.73 |
| 94 | 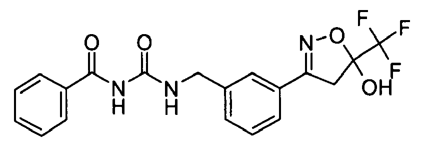 | 407.35 | 407.99 | 8.75 |
| 95 | 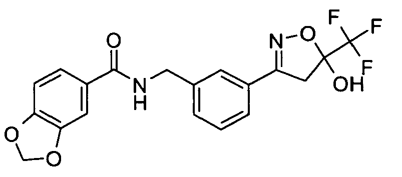 | 408.34 | 408.99 | 8.41 |
| 96 | 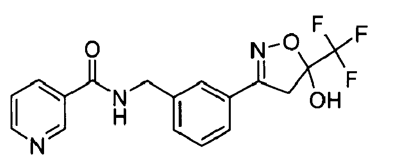 | 365.31 | 366.02 | 6.53 |
| 97 | 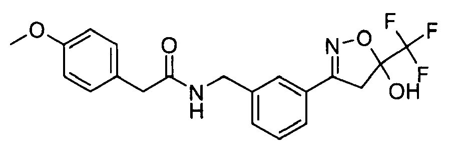 | 408.38 | 408.98 | 8.43 |
| 98 | 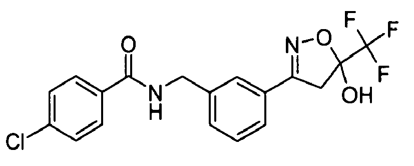 | 398.77 | 398.93 | 9.11 |
| 99 | 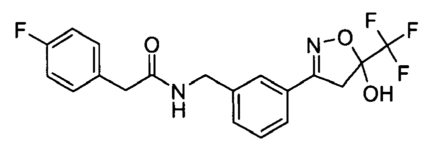 | 396.34 | 396.97 | 8.50 |
| 100 | 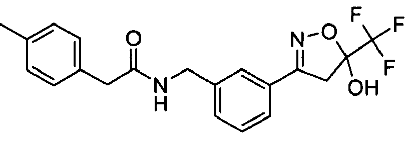 | 392.38 | 392.95 | 8.85 |
| 101 | 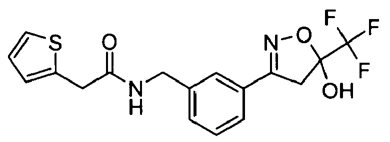 | 384.38 | 384.94 | 7.60 |
| 102 | 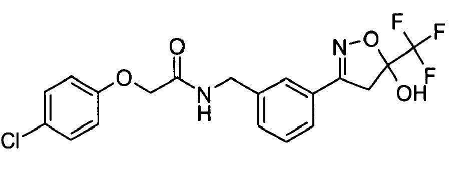 | 428.80 | 428.90 | 9.26 |
| 103 | 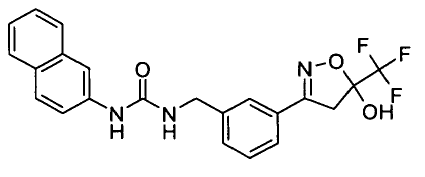 | 429.40 | 429.93 | 9.38 |
| 104 | 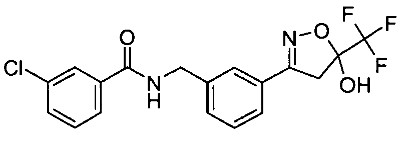 | 398.77 | 398.88 | 8.22 |
| 105 | 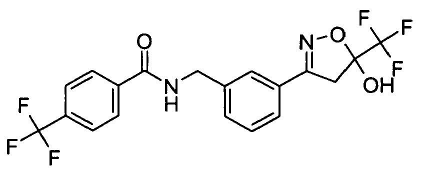 | 432.33 | 432.90 | 9.40 |
| 106 | 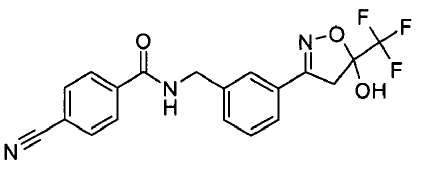 | 389.34 | 389.89 | 8.43 |
| 107 | 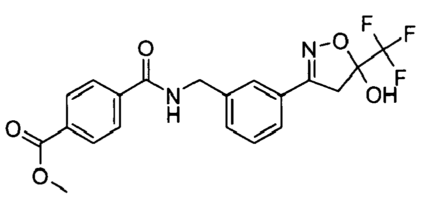 | 422.36 | 422.91 | 8.65 |
| 108 | 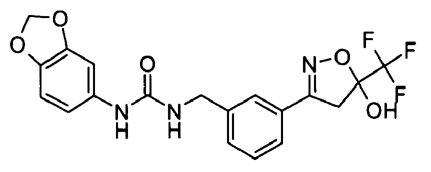 | 423.35 | 423.90 | 7.60 |
| 109 | 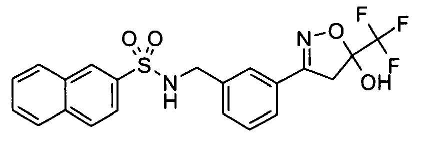 | 450.44 | 450.88 | 9.49 |
| 110 | 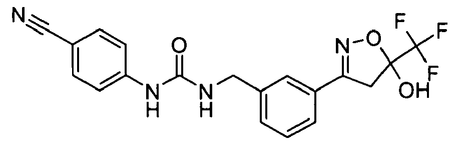 | 404.35 | 404.93 | 8.50 |
| 111 | 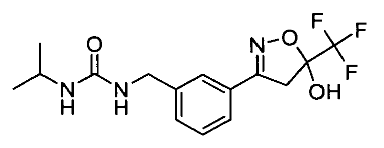 | 345.32 | 346.01 | 7.70 |
| 112 | 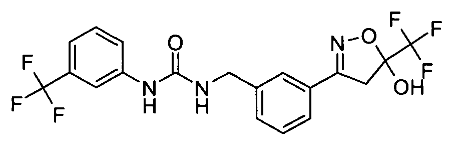 | 447.34 | 447.86 | 8.47 |
| 113 | 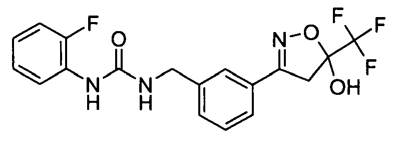 | 397.33 | 397.94 | 8.80 |
| 114 | 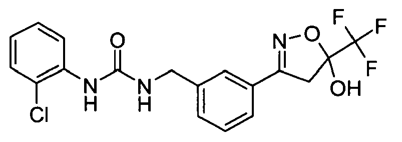 | 413.79 | 413.87 | 9.36 |
| 115 | 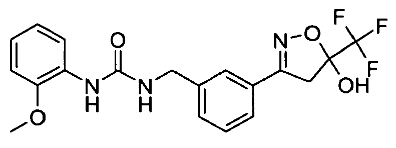 | 409.37 | 409.96 | 8.77 |
| 116 | 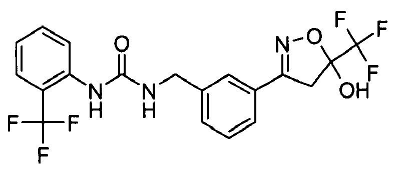 | 447.34 | 447.92 | 8.11 |
| 117 | 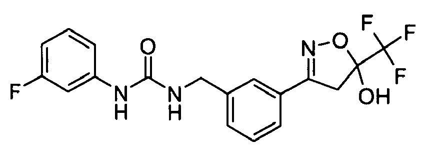 | 397.33 | 397.94 | 8.80 |
| 118 | 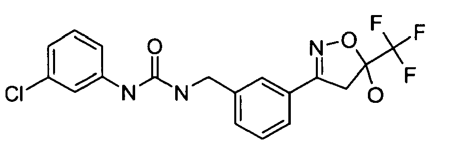 | 413.79 | 413.91 | 9.11 |
| 119 | 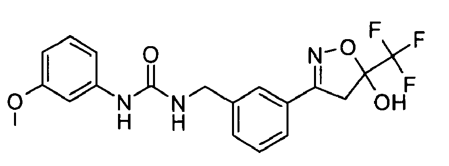 | 409.37 | 410.00 | 8.53 |
| 120 | 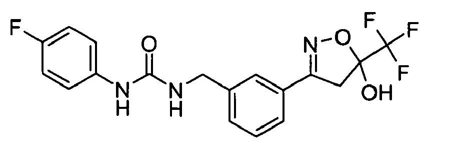 | 397.33 | 397.98 | 7.76 |
| 121 | 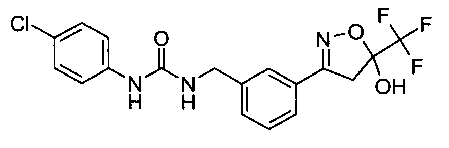 | 413.79 | 413.92 | 9.11 |
| 122 | 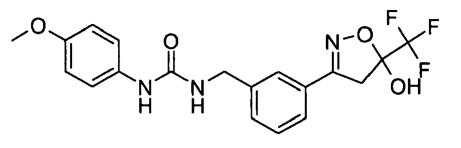 | 409.37 | 410.00 | 8.27 |
| 123 | 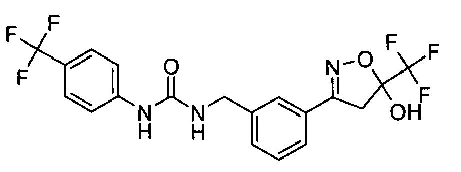 | 447.34 | 447.93 | 9.45 |
| 124 | 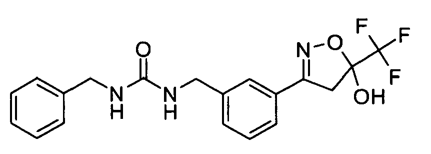 | 393.37 | 394.02 | 8.30 |
| 125 | 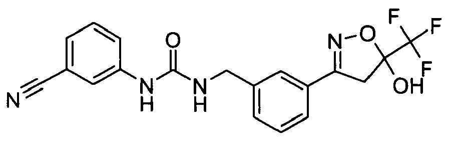 | 404.35 | 405.01 | 8.53 |
| 126 | 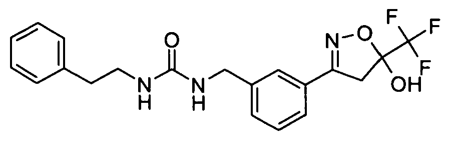 | 407.40 | 408.01 | 8.50 |
| 127 | 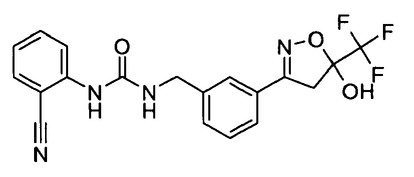 | 404.35 | 404.96 | 6.20 |
| 128 | 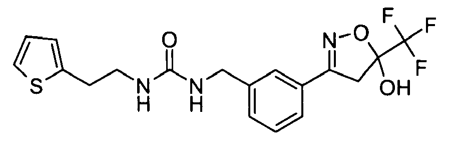 | 413.42 | 413.96 | 8.40 |
| 129 | 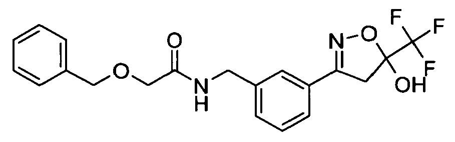 | 408.38 | 409.01 | 8.80 |
| 130 | 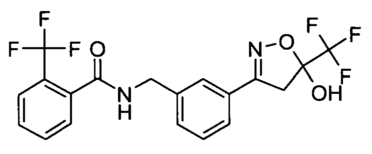 | 432.33 | 432.96 | 8.73 |
| 131 | 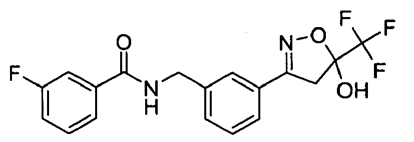 | 382.32 | 383.00 | 8.72 |
| 132 | 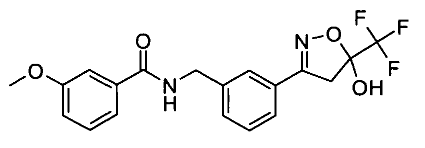 | 394.35 | 395.01 | 8.58 |
| 133 | 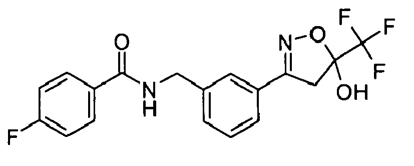 | 382.32 | 382.99 | 8.58 |
| 134 | 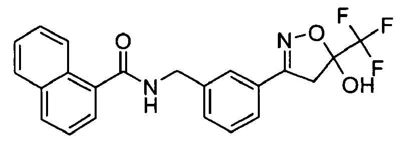 | 414.39 | 415.01 | 9.08 |
| 135 | 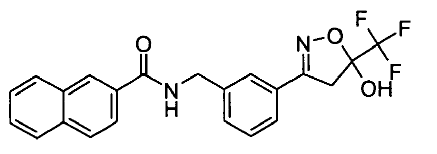 | 414.39 | 415.00 | 9.30 |
| 136 | 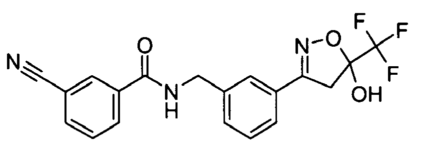 | 389.34 | 390.02 | 8.43 |
| 137 | 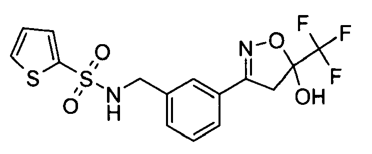 | 406.41 | 406.93 | 8.70 |
| 138 | 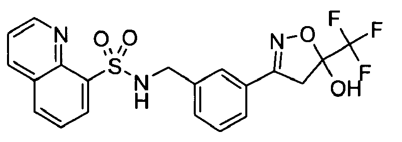 | 451.43 | 451.93 | 8.65 |
| 139 | 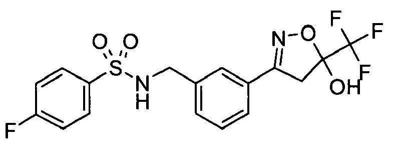 | 418.37 | 418.94 | 8.83 |
| 140 | 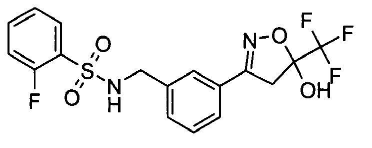 | 418.37 | 418.95 | 8.71 |
| 141 | 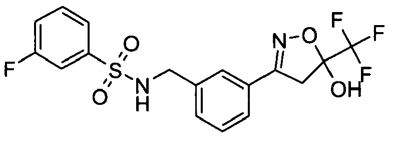 | 418.37 | 418.96 | 9.90 |
| 142 | 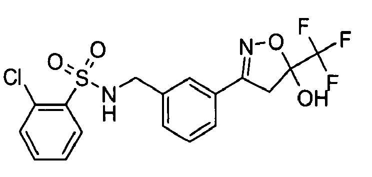 | 434.82 | 434.91 | 8.95 |
| 143 | 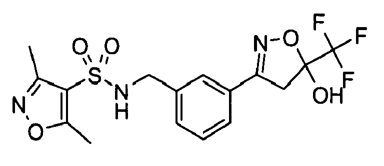 | 419.38 | 419.94 | 8.53 |
| 144 | 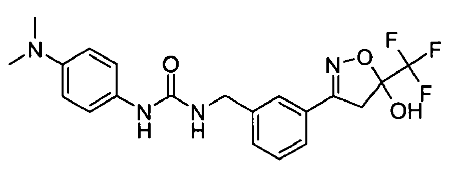 | 422.41 | 423.02 | 7.57 |
| 145 | 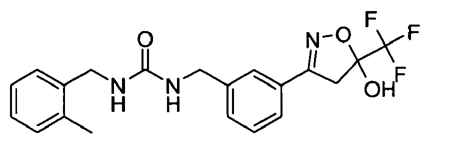 | 407.40 | 408.03 | 8.61 |
| 146 | 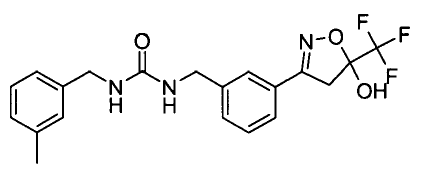 | 407.40 | 408.02 | 8.61 |
| 147 | 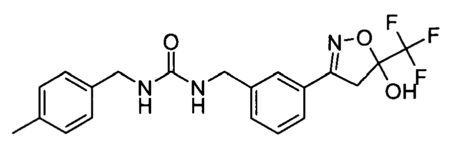 | 407.40 | 408.05 | 8.61 |
| 148 | 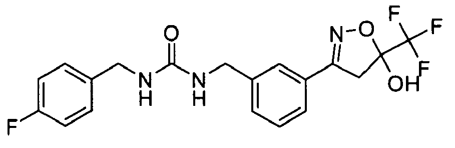 | 411.36 | 412.01 | 9.53 |
| 149 | 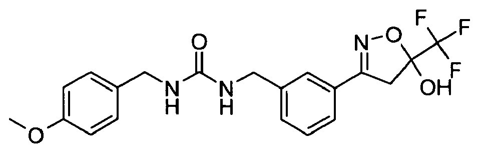 | 423.40 | 424.01 | 8.27 |
| 150 | 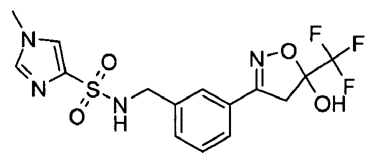 | 404.37 | 404.96 | 7.12 |
| 151 | 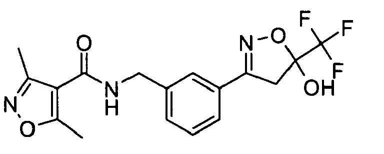 | 383.33 | 384.01 | 9.17 |
| 152 | 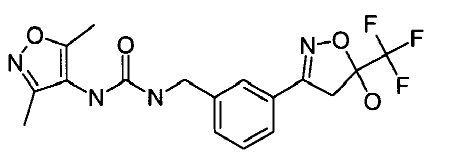 | 398.34 | 399.03 | 7.40 |
| 153 | 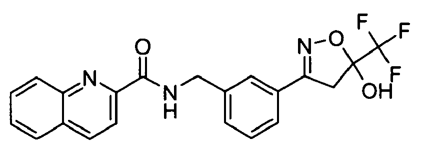 | 415.38 | 415.98 | 10.35 |
| 154 | 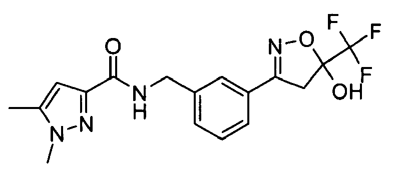 | 382.35 | 383.02 | 7.82 |
| 155 | 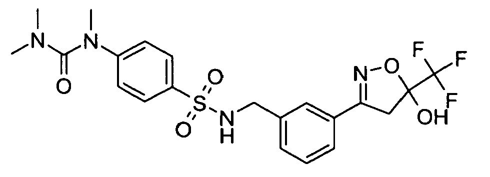 | 500.50 | 500.95 | 8.10 |
| 156 | 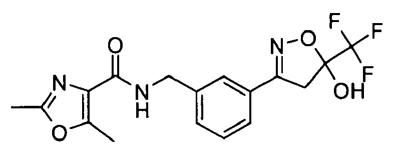 | 383.33 | 384.01 | 9.50 |
| 157 | 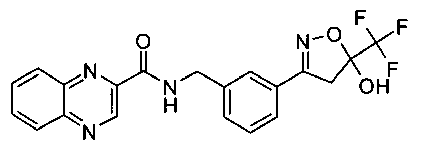 | 416.36 | 416.98 | 9.90 |
| 158 | 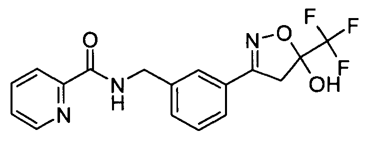 | 365.31 | 366.02 | 8.22 |
| 159 | 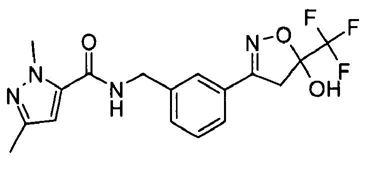 | 382.35 | 383.02 | 7.81 |
| 160 | 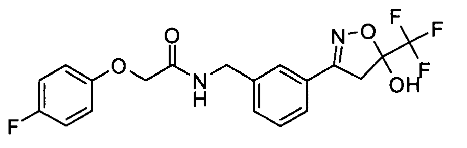 | 412.34 | 412.98 | 9.92 |
| 161 | 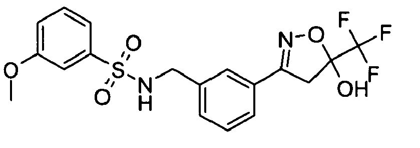 | 430.41 | 430.97 | 8.86 |
| 162 | 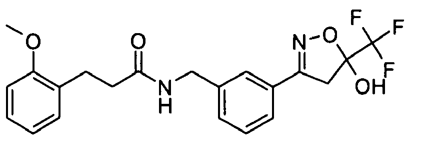 | 422.41 | 423.02 | 8.83 |
| 163 | 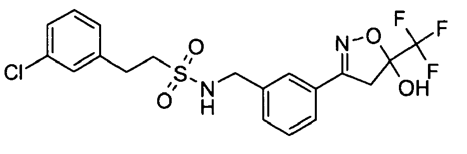 | 462.88 | 462.92 | 10.52 |
| 164 | 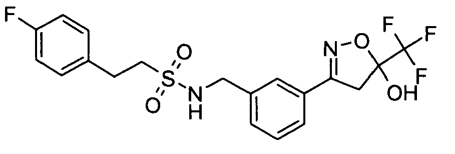 | 446.42 | 446.96 | 9.18 |
| 165 | 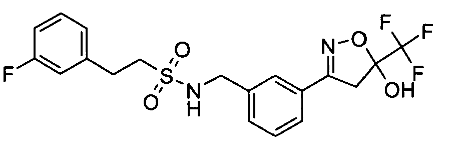 | 446.42 | 446.98 | 9.17 |
| 166 | 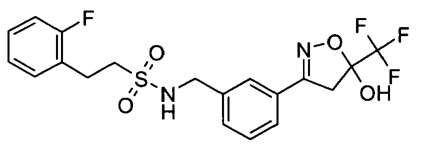 | 446.42 | 446.97 | 9.14 |
| 167 | 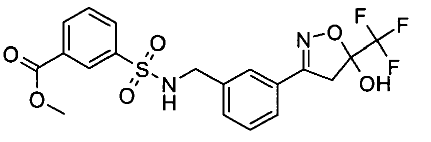 | 458.42 | 458.94 | 9.98 |

Further, the present invention thus provides a method of preparing a compound of general formula (I) *supra,* said method comprising the step of allowing an intermediate amine compound of general formula A : 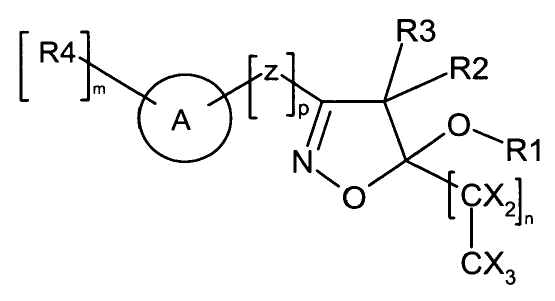 in which R4 is -CH₂NH₂ and m is 1, the remaining definitions being as given *supra,*
to react with a compound of formula B : 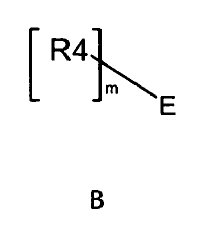 wherein
- R4: represents a substituent selected from the group comprising, preferably consisting of C₁-C₆-alkyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a}, -C(=O)N(R^{a})₂,-C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said C₁-C₆-alkyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a}, -C(=O)N(R^{a})₂,-C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5, wherein R^{a} and R5 are defined *supra* ;
to provide a compound of general formula I : 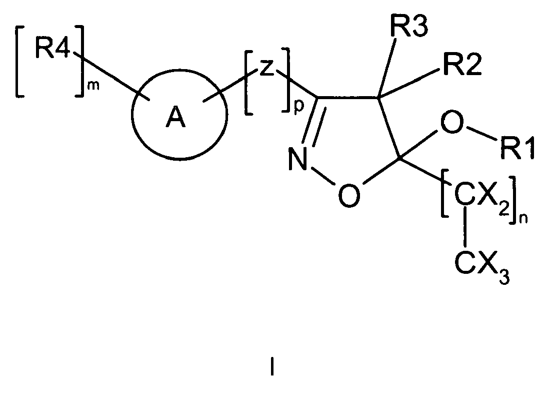 in which formula I, the definitions of A, R1, R2, R3, R4, X, Z, m, n, and p, are given for the main aspect, *supra,* and in which formulae A and B, the definitions of A, R1, R2, R3, X, Z, m, n, and p, are given for each case *supra,* and in which formula B, E is a -C(0)H group. For example, specifically, without the invention being limited thereto :

### 2. Secondary Amines

To the amine of formula A (0.10 mmol), the aldehyde of formula B (0.20 mmol) in tetrahydrofuran (0.5 mL) and sodium triacetoxyborohydride (0.12 mmol) in tetrahydrofuran (0.5 mL) were successively added, and the mixture was stirred at room temperature for 12 h. For work-up ethyl acetate (4 mL) and sodium hydroxide solution (10% in water) were successively added, the mixture was extracted and the organic layer was isolated and concentrated. The residue was purified using preparative HPLC-MS and the products were characterized using analytical HPLC-MS (column LiChroCart Purospher Star 125-4 (125x4.5 mm, RP18e, 5 µm); gradient 5-95% acetonitrile (0.1% trifluoroacetic acid) in water (0.1% trifluoroacetic acid) (10 min.); flow rate 1.2 mL/min; MS ES+).

In accordance with this method, the following compounds were prepared :

| **Example** | **Structure** | **MW** | **Mol. peak** | **RT (min)** |
|---|---|---|---|---|
| 168 | 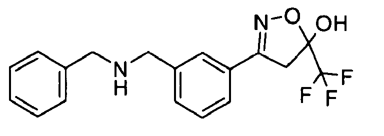 | 350.34 | 351.05 | 6.82 |
| 169 | 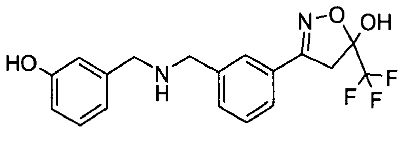 | 366.34 | 367.05 | 6.30 |
| 170 | 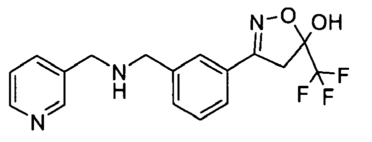 | 351.33 | 352.06 | 5.35 |
| 171 | 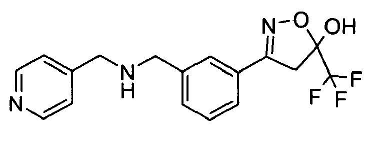 | 351.33 | 352.06 | 5.10 |
| 172 | 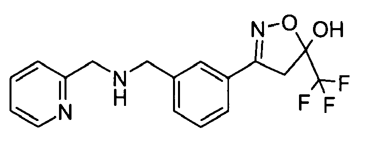 | 351.33 | 352.06 | 6.10 |
| 173 | 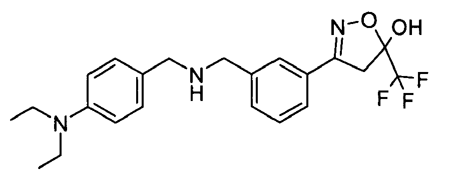 | 421.47 | 422.08 | 5.54 |
| 174 | 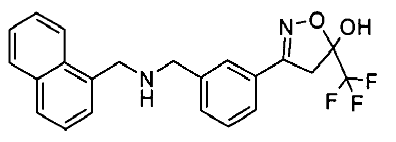 | 400.40 | 401.02 | 7.33 |
| 175 | 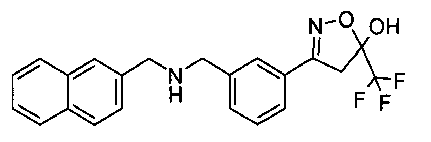 | 400.40 | 401.04 | 7.55 |
| 176 | 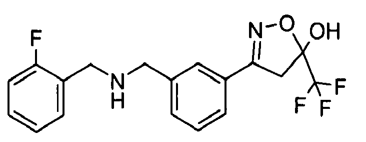 | 368.33 | 369.04 | 6.68 |
| 177 | 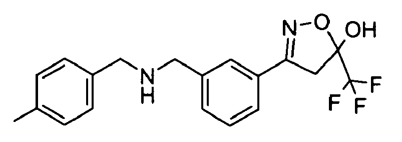 | 364.37 | 365.08 | 7.10 |
| 178 | 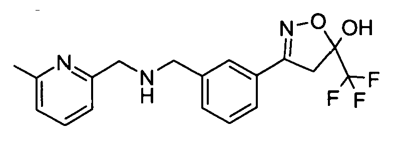 | 365.36 | 366.06 | 6.32 |
| 179 | 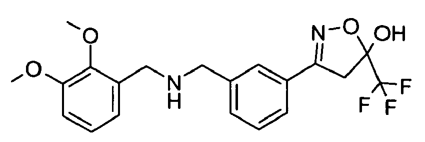 | 410.40 | 411.04 | 7.05 |
| 180 | 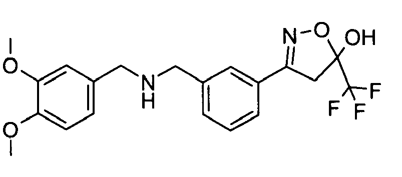 | 410.40 | 411.05 | 6.58 |
| 181 | 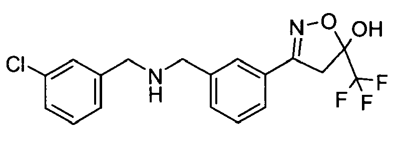 | 384.79 | 385.01 | 7.10 |
| 182 | 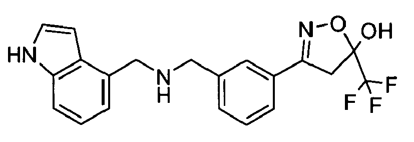 | 389.38 | 390.05 | 6.78 |
| 183 | 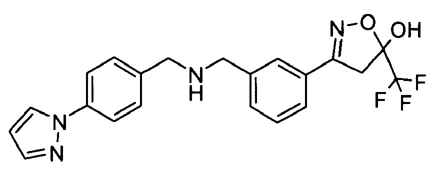 | 416.41 | 417.04 | 6.78 |
| 184 | 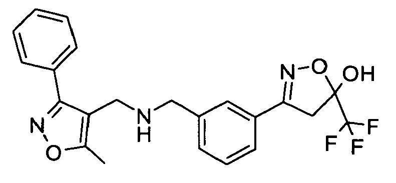 | 431.42 | 432.02 | 7.01 |
| 185 | 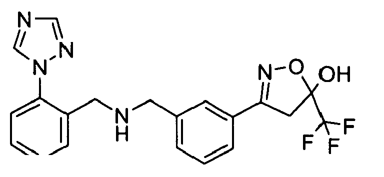 | 417.39 | 418.05 | 6.18 |
| 186 | 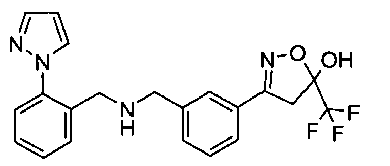 | 416.41 | 417.04 | 7.18 |
| 187 | 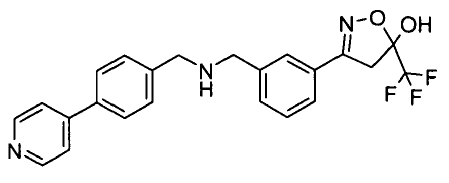 | 427.43 | 428.02 | 5.35 |
| 188 | 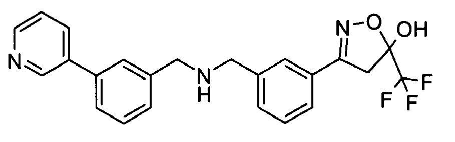 | 427.43 | 428.05 | 5.46 |
| 189 | 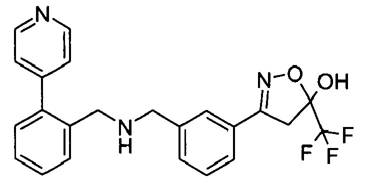 | 427.43 | 428.05 | 5.43 |
| 190 | 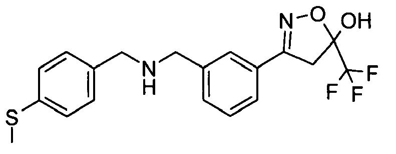 | 396.43 | 397.02 | 7.18 |
| 191 | 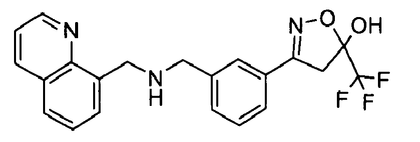 | 401.39 | 402.06 | 6.98 |
| 192 | 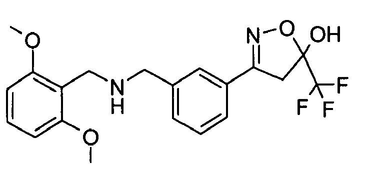 | 410.40 | 411.07 | 7.15 |
| 193 | 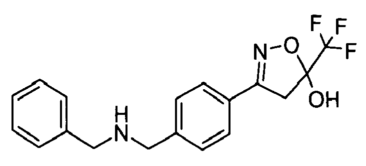 | 350.34 | 351.07 | 6.67 |
| 194 | 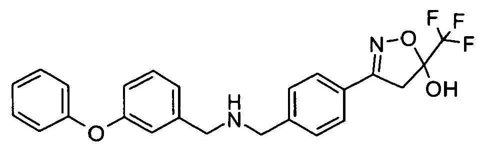 | 442.44 | 443.03 | 7.76 |
| 195 | 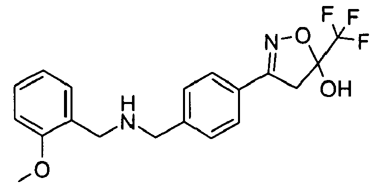 | 380.37 | 381.06 | 6.78 |
| 196 | 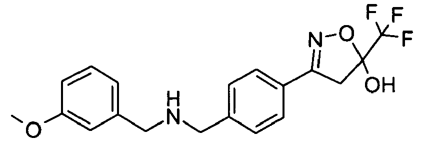 | 380.37 | 381.06 | 6.85 |
| 197 | 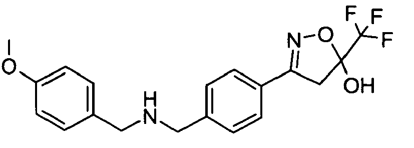 | 380.37 | 381.06 | 6.68 |
| 198 | 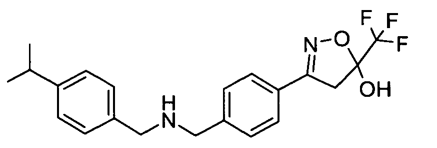 | 392.42 | 393.07 | 7.65 |
| 199 | 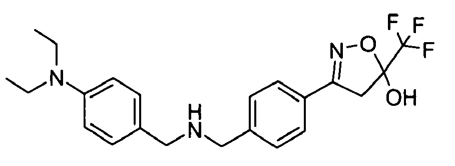 | 421.47 | 422.09 | 5.48 |
| 200 | 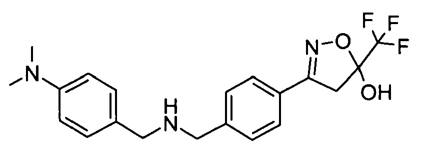 | 393.41 | 394.17 | 6.10 |
| 201 | 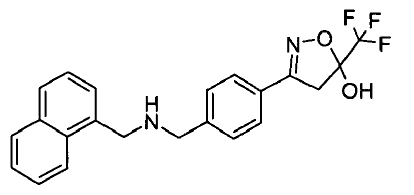 | 400.40 | 401.06 | 7.23 |
| 202 | 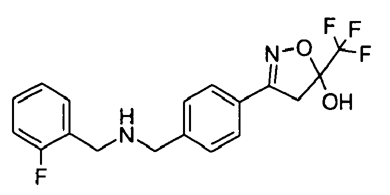 | 368.33 | 369.04 | 6.53 |
| 203 | 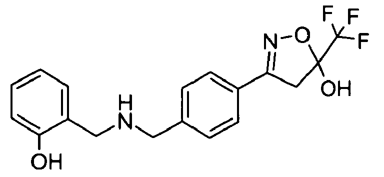 | 366.34 | 367.03 | 6.37 |
| 204 | 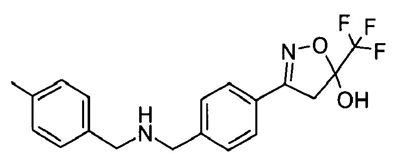 | 364.37 | 365.08 | 7.08 |
| 205 | 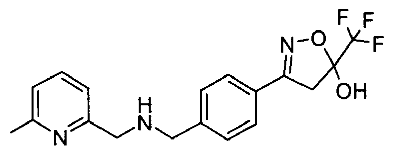 | 365.36 | 366.09 | 6.23 |
| 206 | 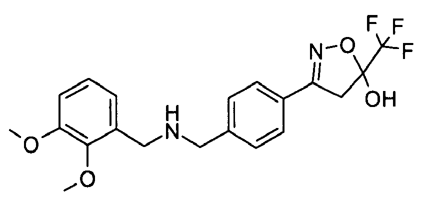 | 410.40 | 411.07 | 6.79 |
| 207 | 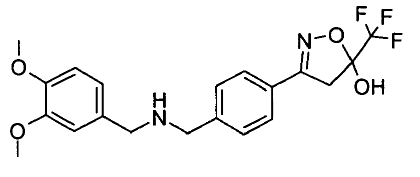 | 410.40 | 411.07 | 6.48 |
| 208 | 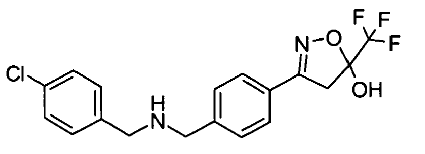 | 384.79 | 385.02 | 7.08 |
| 209 | 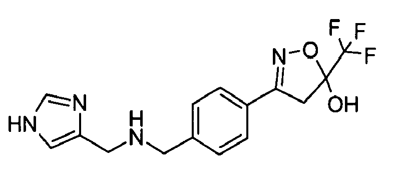 | 340.31 | 341.08 | 4.51 |
| 210 | 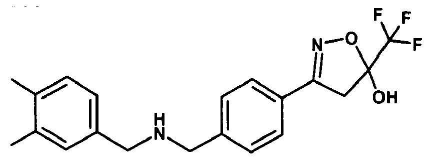 | 378.40 | 379.09 | 7.26 |
| 211 | 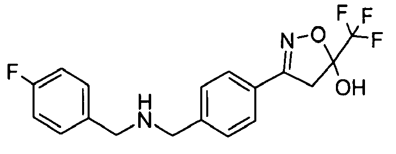 | 368.33 | 369.04 | 6.67 |
| 212 | 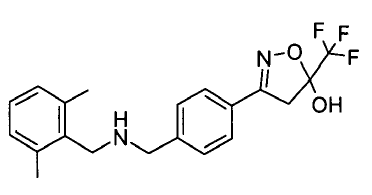 | 378.40 | 379.10 | 7.18 |
| 213 | 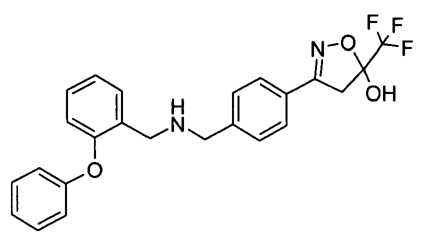 | 442.44 | 443.03 | 7.75 |
| 214 | 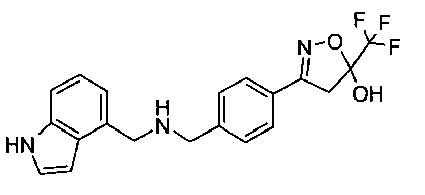 | 389.38 | 390.05 | 6.65 |
| 215 | 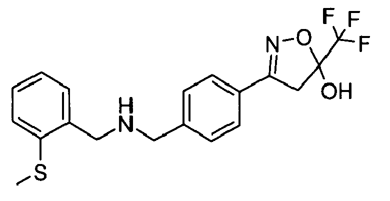 | 396.43 | 397.04 | 6.97 |
| 216 | 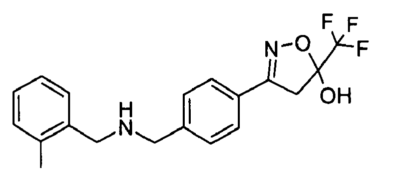 | 364.37 | 365.08 | 6.83 |
| 217 | 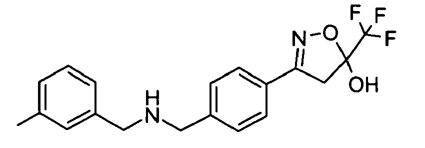 | 364.37 | 365.08 | 6.95 |
| 218 | 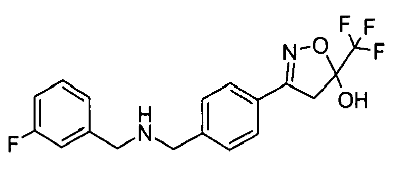 | 368.33 | 369.04 | 6.85 |
| 219 | 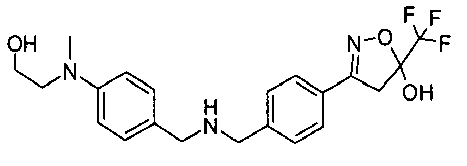 | 423.44 | 424.08 | 5.73 |
| 220 | 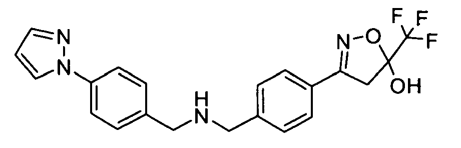 | 416.41 | 417.04 | 6.68 |
| 221 | 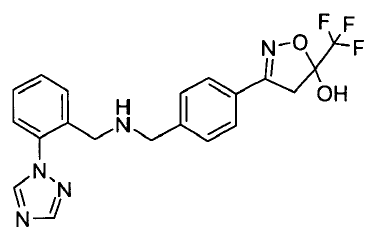 | 417.39 | 418.05 | 6.23 |
| 222 | 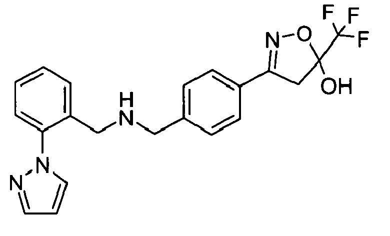 | 416.41 | 417.04 | 7.07 |
| 223 | 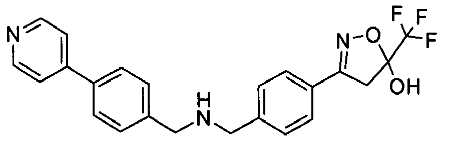 | 427.43 | 428.05 | 5.21 |
| 224 | 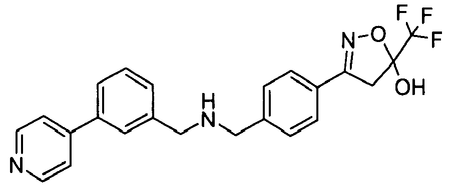 | 427.43 | 428.05 | 5.23 |
| 225 | 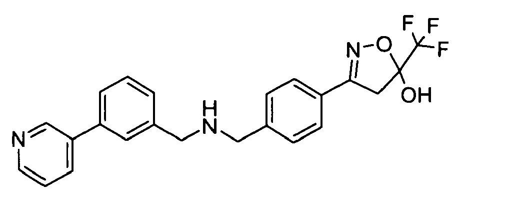 | 427.43 | 428.05 | 5.62 |
| 226 | 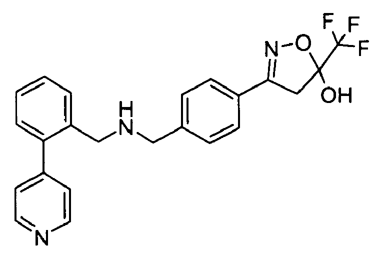 | 427.43 | 428.05 | 5.47 |
| 227 | 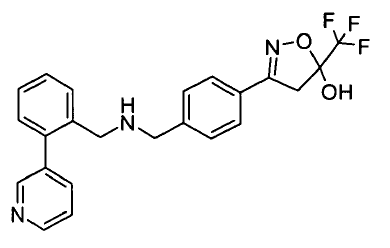 | 427.43 | 428.05 | 5.61 |
| 228 | 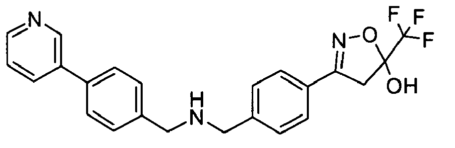 | 427.43 | 428.05 | 5.37 |
| 229 | 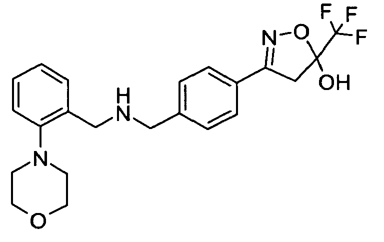 | 435.45 | 436.05 | 6.78 |
| 230 | 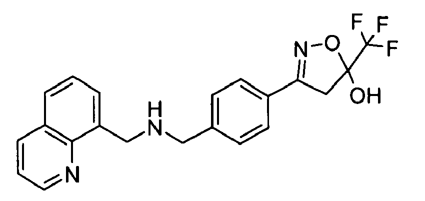 | 401.39 | 402.04 | 6.90 |
| 231 | 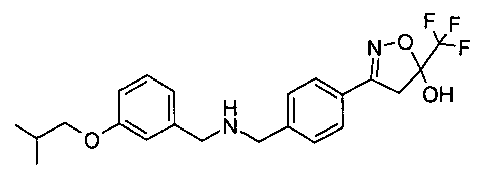 | 422.45 | 423.08 | 8.00 |
| 232 | 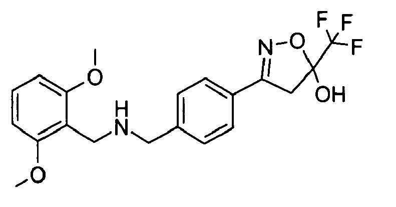 | 410.40 | 411.07 | 7.08 |

### Additional Individual Analogues:

### Example 233 : 3-{4-[(4-Methylsulfanyl-benzylamino)-methyl]-phenyl}-5-trifluoromethyl-4,5-dihydro-isoxazot-5-ol

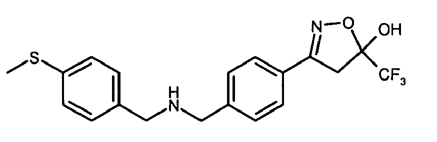

Sodium triacetoxyborohydride (102 mg, 0.48 mmol) was added to a suspension of 3-(4-Aminomethyl-phenyl)-5-trifluoromethyl-4,5-dihydro-isoxazol-5-ol (100 mg, 0.38 mmol) and 4-Methylsulfanyl-benzaldehyde (0.048 ml, 0.36 mmol) in chloroform (5 ml). The reaction mixture was stirred for 4h, washed with a saturated aqueous solution of sodium hydrogencarbonate and extracted with DCM. The organic phase was separated, washed with brine, dried (Na₂SO₄), filtered and concentrated *in vacuo* and the residue purified by flash column chromatography (hexane/ethyl acetate) to give 3-{4-[(4-Methylsulfanyl-benzylamino)-methyl]-phenyl}-5-trifluoromethyl-4,5-dihydro-isoxazol-5-ol (45 mg, 30% yield).
¹H-NMR (DMSO-d6): δ = 3.27 (s, 3H); 3.51 (d, 1 H); 3.60 (s, 2H); 3.68 (s, 2H); 3.89 (d, 1 H); 7.18 (d, 2H); 7.26 (d, 2H); 7.41 (d, 2H); 7.63 (d, 2H) ppm.

### Example 234: [4-(5-Hydroxy-5-trifluoromethyl-4,5-dihydro-isoxazol-3-yl)-benzyl]-carbamic acid pyridin-3-ylmethyl ester

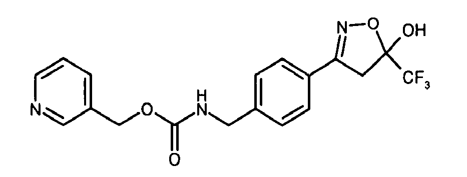

A solution of 3-(Hydroxymethyl)pyridine (0.048 ml, 0.50 mmol) in THF (1 ml) was added dropwise to a suspension of N,N-carbonyldiimidazole (81 mg, 0.50 mmol) in THF (3 ml) at 10°C. After stirring at room temperature for 1h, the reaction mixture was added dropwise to a solution of 3-(4-Aminomethyl-phenyl)-5-trifluoromethyl-4,5-dihydro-isoxazol-5-ol (130 mg, 0.50 mmol), 1,8-Diazabicyclo[5.4.0]undec-7-ene (0.075 ml, 0.50 mmol) and triethylamine (0.069 ml, 0.50 mmol) in THF (2 ml) and stirred overnight. The reaction mixture was concentrated in vacuo and purified by flash column chromatography.(hexane/ethyl acetate to MeOH/ethyl acetate) to give [4-(5-Hydroxy-5-trifluoromethyl-4,5-dihydro-isoxazol-3-yl)-benzyl]-carbamic acid pyridin-3-ylmethyl ester (143 mg, 72% yield).
¹H-NMR (DMSO-d6): δ = 3.51 (d, 1H); 3.89 (d, 1H); 4.23 (d, 2H); 5.05 (s, 2H); 7.33 (d, 2H); 7.47 (q, 1 H); 7.65 (d, 2H); 7.76 (d, 1 H); 7.92 (t, 1 H); 8.51 (m, 1 H); 8.57 (s, 1 H) ppm.

### Example 235 : 4-Dimethylamino-N-[4-(5-hydroxy-5-trifluoromethyl-4,5-dihydro-isoxazol-3-yl)-benzyl]-benzamide

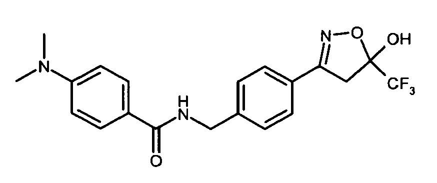

4-Dimethylamino-benzoic acid (48 mg, 0.29 mmol), followed by DMAP (4 mg, 0.033 mmol) and then EDCI (57 mg, 0.30 mmol) was added to a solution of 3-(4-Aminomethyl-phenyl)-5-trifluoromethyl-4, 5-dihydro-isoxazol-5-ol (70 mg, 0.27 mmol) in DCM (1.35 ml) and DMF (0.35 ml). After stirring for 16h, the reaction mixture was quenched with a saturated aqueous solution of ammonium chloride and extracted with ethyl acetate. The organic phase was separated, washed with brine, dried (Na₂SO₄), filtered, concentrated *in vacuo* and the residue purified by flash column chromatography (hexane/ethyl acetate) to give 4-Dimethylamino-N-[4-(5-hydroxy-5-trifluoromethyl-4,5-dihydro-isoxazol-3-yl)-benzyl]-benzamide (25 mg, 24% yield).
¹H-NMR (CDCl₃/CD₃OD 4/1): δ = 2.98 (s, 6H); 3.38 (d, 1H); 3.62 (d, 1H); 4.54 (s, 2H); 6.73 (d, 2H); 7.36 (d, 2H); 7.55 (d, 2H); 7.68 (d, 2H) ppm.

### Example 236 : 3-(4-Hydroxy-phenyl)-5-trifluoromethyl-4,5-dihydro-isoxazol-5-ol

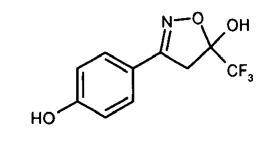

Trifluoroacetic acid (2.5 ml) was added to pentamethylbenzene (317 mg, 2.14 mmol) and 3-(4-Benzyloxy-phenyl)-5-trifluoromethyl-4,5-dihydro-isoxazol-5-ol (180 mg, 0.53 mmol). After stirring for 15h, the reaction mixture was concentrated in vacuo and the residue washed with hexane and dried to give 3-(4-Hydroxy-phenyl)-5-trifluoromethyl-4,5-dihydro-isoxazol-5-ol (114 mg, 86% yield).
¹H-NMR (acetone-d6): δ = 3.53 (d, 1H); 3.86 (d, 1H); 6.93 (d, 2H); 7.61 (d, 2H) ppm.

### Example 237 : 2-[4-(5-Hydroxy-5-trifluoromethyl-4,5-dihydro-isoxazol-3-yl)-phenoxy]-N-phenyl-acetamide

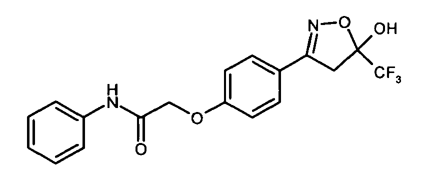

Cesium carbonate (56 mg, 0.17 mmol) was added to a solution of 3-(4-Hydroxy-phenyl)-5-triftuoromethyl-4,5-dihydro-isoxazol-5-ol (21 mg, 0.085 mmol) in DMF (1.6 ml). The reaction mixture was cooled to 0°C and 2-Chloro-N-phenyl-acetamide (16 mg, 0.094 mmol) was added. After warming to room temperature and stirring for 17h, the reaction mixture was diluted with brine and extracted with ethyl acetate. The organic phase was separated, washed with water then brine, dried (Na₂SO₄), filtered and concentrated *in vacuo* and the residue purified by flash column chromatography (hexane/ethyl acetate) to give 2-[4-(5-Hydroxy-5-trifluoromethyl-4,5-dihydro-isoxazol-3-yl)-phenoxy]-N-phenyl-acetamide (15 mg, 46% yield).
¹H-NMR (acetone-d6): δ = 3.58 (d, 1H); 3.90 (d, 1H); 4.74 (s, 2H); 7.04-7.15 (m, 3H); 7.28-7.34 (m, 3H); 7.67-7.75 (m, 4H); 9.33 (s, 1H) ppm.

### Example 238 : Benzenesulfonic acid 4-(5-hydroxy-5-trifluoromethyl-4,5-dihydro-isoxazol-3-yl)-phenyl ester

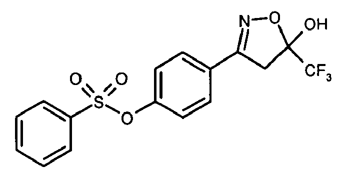

Triethylamine (0.027 ml, 0.19 mmol) was added to a suspension of 3-(4-Hydroxyphenyl)-5-trifluoromethyl-4,5-dihydro-isoxazol-5-ol (43 mg, 0.17 mmol) in DCM (2 ml). The reaction mixture was cooled to -5°C and benzene sulfonyl chloride (0.024 ml, 0.19 mmol) was added. After warming to room temperature and stirring for 4h, the reaction mixture was quenched with a saturated aqueous solution of ammonium chloride and extracted with ethyl acetate. The organic phase was separated, washed with brine, dried (Na₂SO₄), filtered and concentrated *in vacuo* to give Benzenesulfonic acid 4-(5-hydroxy-5-trifluoromethyl-4,5-dihydro-isoxazol-3-yl)-phenyl ester (67 mg, 99% yield).
¹H-NMR (acetone-d6): δ = 3.60 (d, 1H); 3.95 (d, 1H); 7.14 (d, 2H); 7.44 (s, 1H); 7.63-7.93 (m, 7H) ppm.

### BIOLOGICAL DATA

### HDAC6 assay

HDAC6 inhibitory activity of compounds of the present invention was quantified employing the HDAC6 assay as described in the following paragraphs.

Recombinant full-length human HDAC6 with C-terminal His-tag was expressed in Sf-9 insect cells and purified using Nickel-NTA chromatography. The assay was performed using the HDAC-assay-kit purchased from Biomol (Hamburg, Germany). HDAC6 catalyzes the deacetylation of the FluordeLys substrate (purchased from Biomol #KI-104). This sensitizes the substrate to the developer which then generates a fluorophore. This fluorophore is excited with 360 nm light and the emitted light is detected on a fluorometric plate reader at 460 nm.

HDAC6 was incubated for 90 min at 22°C in the presence of different concentrations of test compounds in 15 µl assay buffer [25 mM Tris/HCl pH 7.5, 10 mM KCl, 0.04% NP40, 0.2% BSA, 22.5 µM Fluor de Lys substrate, 1% DMSO]. The concentration of HDAC6 was adjusted depending of the activity of the enzyme lot and was chosen appropriate to have the assay in the linear range, typical concentrations were in the range of 20 nM. The reaction was stopped by the addition of 5 µl of a Stop/Developer solution [25 mM Tris/HCl pH 7.5, 1:20 diluted 20x Developer solution (purchased from Biomol #Kl- 105), 1 µM Trichostatin A].

The resulting mixture was incubated for 1 h at 22°C to allow the developing reaction to proceed. Subsequently the amount of deacetylated substrate was evaluated on a fluorometric plate reader (e.g. Acquest, molecular Devices) by measurement of the emission at 460 nm after excitation with 360 nm light. The data were normalised (enzyme reaction without inhibitor = 0 % inhibition, all other assay components but no enzyme = 100 % inhibition) and IC50 values were calculated by a 4 parameter fit using an inhouse software.

The compounds of the present invention display IC50 values in the above-mentioned HDAC6 assay of less than 100 mM.

### BIBLIOGRAPHIC REFERENCES

1. GREENLEE RT, MURRAY T, BOLDEN S, WINGO PA: Cancer Statistics 2000. CA Cancer J. Clin. (2000) 50: 7-33.
2. MAI A, MASSA A, ROTILI D, CERBARA I, VALENTE S, PEZZI R, SIMEONI S, RAGNO R: Histone deacetylation in epigenetics: An attractive target for anticancer therapy. Med. Res. Rev. (2005) 25: 261-309.
3. MCLAUGHLIN F, LA THANGUE N: Histone deacetylase inhibitors open new doors in cancer therapy. Biochem. Pharmacol. (2004) 68: 1139-1144.
4. MEI S, HO AD, MAHLKNECHT U: Role of histone deacetylase inhibitors in the treatment of cancer. International Journal of Oncology (2004) 25: 1509-1519.
5. BHALLA KN: Epigenetic and chromatin modifiers as targeted therapy of hematologic malignancies. J. Clin. Oncol. (2005) 23(17): 3971-3993.
6. ROSATO R, GRANT S: Histone deacetylase inhibitors in clinical development. Expert. Opin. Invest. Drugs (2004) 13: 21-38.
7. MARKS PA, RICHON VM, BRESLOW R, RIFKIND RA: Histone deacetylase inhibitors as new cancer drugs. Curr. Opin. Oncol. (2001) 13: 477-483.
8. KELLY WK, O'CONNOR OA, MARKS PA: Histone deacetylase inhibitors: from target to clinical trial. Expert. Opin. Invest. Drugs (2002) 11: 1695-1713.
9. HESS-STUMPP H: Histone deacetylase inhibitors and cancer: from cell biology to the clinic, European Journal of Cell Biology (2005) 84: 109-121.
10. DRUMMOND DC, NOBLE CO, KIRPOTIN DB, GUO Z, SCOTT GK, BENZ CC: Clinical Development of Histone Deacetylase Inhibitors as Anticancer Agents. Annu Rev Pharmacol Toxicol (2005) 45: 495-528.
11. PIEKARTZ R, BATES S: A Review of Depsipeptide and Other Histone Deacetylase Inhibitors in Clinical Trials. Curr. Pharm. Des. (2004) 10(19): 2289-2298.
12. DE RUIJTER AJM, VAN GENNIP AH, CARON HN, KEMP S, VAN KUILENBURG ABP: Histone deacetylases (HDACs): characterization of the classical HDAC family. Biochem. J. (2003) 370: 737-749.
13. GRAY GG, EKSTROM TJ: The human histone deacetylase family. Exp. Cell. Res. (2001) 262: 75-83.
14. HAGGARTY SJ, KOELLER KM, WONG JC, GROZINGER CM, SCHREIBER SL: Domain-selective small molecule inhibitor of histone deacetylase 6 (HDAC6)-mediated tubulin deacetylation. Proc. Natl. Acad. Sci. USA (2003) 100(8): 4389-4394 .
15. BALI P, PRANPAT M, BRADNER J, BALASIS M, FISKUS W, GUO F, ROCHA K, KUMARASWAMY S, BOYAPALLE S, ATADJA P, SETO E, BHALLA K: Inhibition of histone deacetylase 6 acetylates and disrupts the chaperone function of heat shock protein 90: a novel basis for antileukemia activity of histone deacetylase inhibitors. J. Biol. Chem. (2005) 280(29): 26729-26734.
16. GAO L, CUETO MA, ASSELBERGS F, ATAOJA P: Cloning and Functional Characterization of HDAC11, a Novel Member of the Human Histone Deacetylase Family. J. Biol. Chem. (2002) 277: 25748-25755.
17. FINNIN MS, DONIGIAN JR, COHEN A, RICHON VM, RIFKIND, RA, MARKS P, BRESLOW R, PAVLETICH NP: Structures of a histone deacetylase homologue bound to the TSA and SAHA inhibitors. Nature (1999) 401: 188-193.
18. SOMOZA JR, SKENE RJ, KATZ BA, MOL C, HO JD, JENNINGS AJ, LUONG C, ARVAI A, BUGGY JJ, CHI E, TANG J, SANG BC, VERNER E, WYNANDS R, LEAHY EM, DOUGAN DR, SNELL G, NAVRE M, KNUTH MW, SWANSON RV, MCREE DE, TARI LW: Structural Snapshots of Human HDAC8 Provide Insights into the Class I Histone Deacetylases. Structure (2004) 12: 1325-1334.
19. VANNINI A, VOLPARI C, FILOCAMO G, CASAVOLA EC, BRUNETTI M, RENZONI D, CHAKRAVARTY P, PAOLINI C, DE FRANCESCO R, GALLINARI P, STEINKUHLER C, DI MARCO S: Crystal structure of a eukaryotic zinc-dependent histone deacetylase, human HDAC8, complexed with a hydroxamic acid inhibitor. Proceedings of the National Academy of the United States (2004) 101: 15064-15069.
20. YANG K, LOU B: Molecular Diversity of Hydroxamic Acids: Part I. Solution- and Solid-Phase Synthesis. Mini Reviews in Med. Chem. (2003) 3: 349-360.
21. LOU B, YANG K: Molecular Diversity of Hydroxamic Acids: Part II. Potential Therapeutic Applications. Mini Reviews in Med. Chem. (2003) 3: 609-620.
22. CURTIN ML: Synthesis of novel hydroxamate and non-hydroxamate histone deacetylase inhibitors. Curr. Opin. Drug Disc. Dev. (2004) 7: 848-868.
23. SHABBEER S, CARDUCCI MA: Focus on deacetylation for therapeutic benefit. Investigational Drugs (2005) 8: 144-154.
24. KELLY WK, CONNOR OA, KRUG L, CHIAO J, HEANEY M, CURLEY T: Phase I Study of the Oral Histone Deacetylase Inhibitor, Suberoylanilide Hydroxamic Acid (SAHA), in Patients with Advanced Cancer. J. Clin. Oncol. (2005) 23(17): 3923-3931 .
25. KELLY WK, MARKS PA: Drug Insight: Histone Deacetylase Inhibitors-Development of the New Targeted Anticancer agent Suberoylanilide Hydroxamic Acid. Nature Clinical Practice Oncology. (2005) 2(3): 150-157.
26. MCLAUGHLIN F, LA THANGUE NB: Histone Deacetylase Inhibitors in Psoriasis Therapy, Current Drug Targets - Inflammation & Allergy (2004) 3: 213-219.
27. ARTS J, DE SCHEPER S, VAN EMELEN, K: Histone Deacetylase Inhibitors: From Chromatin Remodeling to Experimental Cancer Therapeutics. Current Med. Chem. (2003) 10: 2343-2350.
28. BERTOS NR, WANG AH, YANG XJ: Class II histone deacetylases: structure, function, and regulation. Biochem Cell Biol. (2001) 79: 243-252.
29. UNGERSTEDT JS, SOWA Y, XU WS, SHAO Y, DOKMANOVIC M, PEREZ G, NGO L, HOLMGREN A, JIANG X, MARKS PA: Role of thioredoxin in the response of normal and transformed cells to histone deacetylase inhibitors. Proc. Natl. Acad. Sci. USA (2005) 102(3): 673-678.

## Claims

1. A compound of general formula (I) : 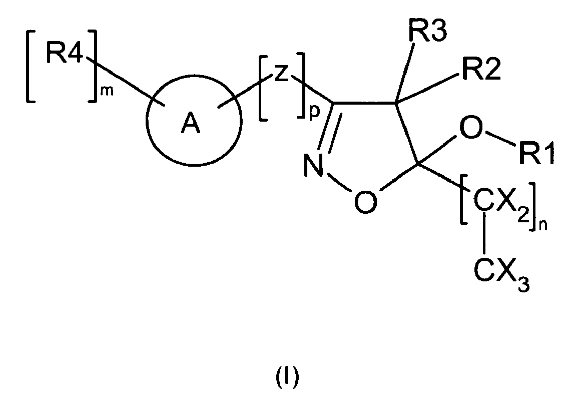 in which :
R1 represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
R2 and R3, independently of each other, represent a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C,-C₆-alkyl-C(=0)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a}, -C(=O)OR^{a}, -C(=O)N(R^{a})2,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})2,-P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})2, -C₁-C₆-alkyl-NR^{a}S(=O)₂R^{a}, -C₁-C₆-alkyl-S(=O)₂R^{a},-C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
or,
together, form a C₃-C₁₀-cycloalkyl or C₃-C₁₀-heterocydoalkyL;
A represents an aryl or heteroaryl group ;
X represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
Z represents a C₂-C₆-alkenyl group, itself being optionally substituted, one or more times, in the same way or differently, with R5 ;
R4 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=0)R^{a},-C(=0)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
R5 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
R^{a} represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
R^{b} represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkytsulfonyl, C₁-C₆-atkytamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂ ;
R^{c} represents, independently from each other, a substituent, which is identical or different, selected from the group comprising, preferably consisting of, a hydrogen, aryl and C₁-C₆-alkyl;
n represents an integer of 0, 1, 2, 3, 4, 5, or 6 ;
m represents an integer of 0, 1, 2, 3, 4, or 5 ; and
p represents an integer of 0, 1, 2, 3, 4, or 5.

2. The compound according to claim 1, wherein :
R1 represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
R2 represents a hydrogen atom ;
R3 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
A represents an aryl or heteroaryl group ;
X represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
Z represents a C₂-C₆-alkenyl group, itself being optionally substituted, one or more times, in the same way or differently, with R5 ;
R4 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-atkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})2 substituent is itself optionally substituted, one or more times, in the same way or differently, with R5 ;
R5 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
R^{a} represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
R^{b} represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂;
R^{c} represents, independently from each other, a substituent, which is identical or different, selected from the group comprising, preferably consisting of, a hydrogen, aryl and C₁-C₆-alkyl ;
n represents an integer of 0, 1, 2, 3, 4, 5, or 6 ;
m represents an integer of 0, 1, 2, 3, 4, or 5 ; and
p represents an integer of 0, 1, 2, 3, 4, or 5.

3. The compound according to claim 1 or 2, wherein :
R1 represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
R2 and R3 represent a hydrogen atom ;
A represents an aryl or heteroaryl group ;
X represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
Z represents a C₂-C₆-alkenyl group, itself being optionally substituted, one or more times, in the same way or differently, with R5 ;
R4 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -F(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsutfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
R5 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
R^{a} represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)_{R}^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
R^{b} represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂;
R^{c} represents, independently from each other, a substituent, which is identical or different, selected from the group comprising, preferably consisting of, a hydrogen, aryl and C₁-C₆-alkyl ;
n represents an integer of 0, 1, 2, 3, 4, 5, or 6 ;
m represents an integer of 0, 1, 2, 3, 4, or 5 ; and
p represents an integer of 0, 1, 2, 3, 4, or 5.

4. The compound according to any one of claims 1 to 3, wherein :
R1 represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂, P(=O)(OR^{a})₂ substituent is itself optionally substituted, one or more times, in the same way or differently, with R5 ;
R2 and R3 represent a hydrogen atom ;
A represents an aryl or heteroaryl group ;
X represents, independently of each other, a substituent selected from the group comprising, preferably consisting of, a hydrogen, fluorine, chlorine, bromine, and iodine atom, wherein at least one X substituent is a fluorine atom ;
wherein : said -(CX₂)ₙCX₃ group is itself optionally substituted, one or more times, in the same way or differently, with R5 ;
R4 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
R5 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
R^{a} represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
R^{b} represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂ ;
R^{c} represents, independently from each other, a substituent, which is identical or different, selected from the group comprising, preferably consisting of, a hydrogen, aryl and C₁-C₆-alkyl ;
n represents an integer of 0, 1, 2, 3, 4, 5, or 6 ;
m represents an integer of 0, 1, 2, 3, 4, or 5 ; and
p represents an integer of 0.

5. The compound according to any one of claims 1 to 4, wherein :
R1 represents a substituent selected from the group comprising, preferably consisting of, a hydrogen, sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a}, -C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, nitro, -C(=O)R^{a},-C(=O)OR^{a}, -C(=O)N(R^{a})₂, -C(=S)N(R^{a})₂, -S(=O)₂R^{a}, -S(=O)₂N(R^{a})₂,-P(=O)(OR^{a})₂ substituent is itself optionally substituted, one or more times, in the same way or differently, with R5 ;
R2 and R3 represent a hydrogen atom ;
A represents an aryl or heteroaryl group ;
X represents, independently of each other, a subsituent selected from the group comprising, preferably consisting of, a hydrogen atom, and a fluorine atom, wherein at least one X substituent is a fluorine atom ;
R4 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂, -C₁-C₆-alkyl-N(R^{a})₂, -C₁-C₆-alkyl-C(=O)R^{a},-C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro,-C(=O)R^{a}, -C(=O)OR^{a}, -C(=O)N(R^{a})₂, -C(=S)N(R^{a})₂, -NR^{a}C(=O)OR^{a},-NR^{a}C(=O)N(R^{a})₂, -NR^{a}C(=O)R^{a}, -NR^{a}C(=S)R^{a}, -OC(=O)N(R^{a})₂,-NR^{a}S(=O)₂R^{a}, -S(=O)₂R^{a}, -S(=O)₂N(R^{a})₂, -P(=O)(OR^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5 ;
R5 represents a substituent selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{a},-C(=O)OR^{a}, -C(=O)N(R^{a})₂, -C(=S)N(R^{a})₂, -NR^{a}C(=O)OR^{a}, -NR^{a}C(=O)N(R^{a})₂, -NR^{a}C(=O)R^{a}, -NR^{a}C(=S)R^{a}, -OC(=O)N(R^{a})₂, -NR^{a}S(=O)₂R^{a}, -S(=O)₂R^{a},-S(=O)₂N(R^{a})₂, -P(=O)(OR^{a})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, -C(=O)R^{a}, -C(=O)OR^{a}, -C(=O)N(R^{a})₂,-C(=S)N(R^{a})₂, -NR^{a}C(=O)OR^{a}, -NR^{a}C(=O)N(R^{a})₂, -NR^{a}C(=O)R^{a},-NR^{a}C(=S)R^{a}, -OC(=O)N(R^{a})₂, -NR^{a}S(=O)₂R^{a}, -S(=O)₂R^{a}, -S(=O)₂N(R^{a})₂,-P(=O)(OR^{a})₂ substituent is itself optionally substituted, one or more times, in the same way or differently with R^{b} ;
R^{a} represents, independently from each other, a substituent, which is identical or different, selected from the group comprising, preferably consisting of, a hydrogen or halogen atom, cyano, hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{b}, -C(=O)OR^{b},-C(=O)N(R^{b})₂, -C(=S)N(R^{b})₂, -NR^{b}C(=O)OR^{b}, -NR^{b}C(=O)N(R^{b})₂,-NR^{b}C(=O)R^{b}, -NR^{b}C(=S)R^{b}, -OC(=O)N(R^{b})₂, -NR^{b}S(=O)₂R^{b}, -S(=O)₂R^{b},-S(=O)₂N(R^{b})₂, -P(=O)(OR^{b})₂ ; wherein said hydroxy, mercapto, sulfenyl, sulfinyl, sulfonyl, amino, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ;
R^{b} represents, independently from each other, a substituent, which is identical or different, selected from the group comprising, preferably consisting of, a hydrogen or halogen atom, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyl, C₁-C₆-alkoxy, C₁-C₆-alkylthio, C₁-C₆-alkylsulfenyl, C₁-C₆-alkylsulfinyl, C₁-C₆-alkylsulfonyl, C₁-C₆-alkylamino, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, aryloxy, heteroaryl, heteroaryloxy, nitro, -C(=O)R^{c},-C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, -NR^{c}C(=S)R^{c}, -OC(=O)N(R^{c})₂, -NR^{c}S(=O)₂R^{c}, -S(=O)₂N(R^{c})₂,-P(=O)(OR^{c})₂;
R^{c} represents, independently from each other, a substituent, which is identical or different, selected from the group comprising, preferably consisting of, a hydrogen, aryl and C₁-C₆-alkyl ;
n represents an integer of 0, 1, 2, 3, 4, 5, or 6 ;
m represents an integer of 0, 1, 2, 3, 4, or 5 ; and
p represents an integer of 0.

6. A method of preparing a compound of general formula (I) according to any one of claims 1 to 5, said method comprising the step of allowing an intermediate amine compound of general formula A : 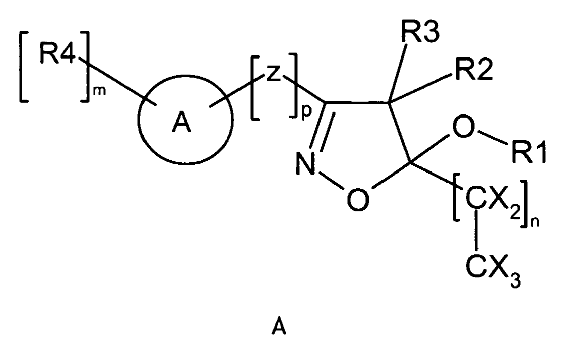 in which R4 is -CH₂NH₂ and m is 1, and in which A, R1, R2, R3, X, Z, m, n, and p, are given in any one of claims 1 to 5,
to react with a compound of formula B : 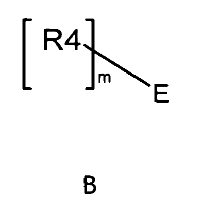 wherein
R4 represents a substituent selected from the group comprising, preferably consisting of sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a},-C(=O)N(R^{a})₂, -C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl(=O)N(R^{a})₂,-C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a},-C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂, wherein said sulfenyl, sulfinyl, sulfonyl, C₁-C₆-alkyl, C₁-C₆-alkylsulfenyl, C₁-C₆-alkylsulfinyl, C₁-C₆-alkylsulfonyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a},-C(=O)OR^{a}, -C(=O)N(R^{a})₂, -C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂, -C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=0)2R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5, wherein R^{a} and R5 are as defined in any one of claims 1 to 5 ; and E is a leaving group ;
to provide a compound of general formula I : 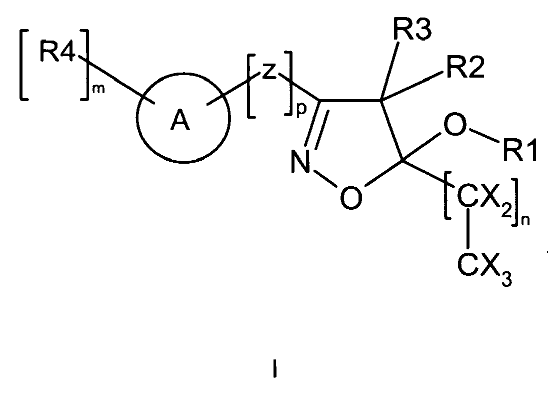 in which formula I, the definitions of A, R1, R2, R3, R4, X, Z, m, n, and p, are given in any one of claims 1 to 5.

7. A method of preparing a compound of general formula (I) according to any one of claims 1 to 5, said method comprising the step of allowing an intermediate amine compound of general formula A : 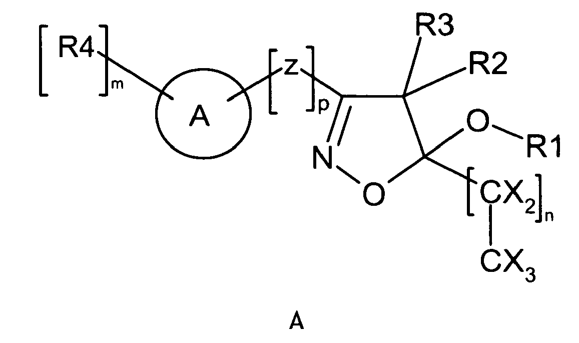 in which R4 is -CH₂NH₂ and m is 1, and in which A, R1, R2, R3, X, Z, m, n, and p, are given in any one of claims 1 to 5,
to react with a compound of formula B : 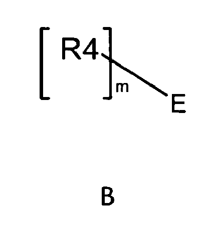 wherein
R4 represents a substituent selected from the group comprising, preferably consisting of C₁-C₆-alkyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a}, -C(=O)N(R^{a})₂,-C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-atkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-R(=O)(OR^{a})₂, wherein said C₁-C₆-alkyl, C₂-C₆-alkenyl, C₂-C₆-alkynyl, C₃-C₁₀-cycloalkyl, C₃-C₁₀-heterocycloalkyl, C₁-C₆-haloalkyl, hydroxy-C₁-C₆-alkyl, aryl, heteroaryl, -C(=O)R^{a}, -C(=O)OR^{a}, -C(=O)N(R^{a})₂,-C(=S)N(R^{a})₂, -S(=O)₂R^{a}, -S(=O)₂N(R^{a})₂, -C₁-C₆-alkyl-N(R^{a})₂, -C₁-C₆-alkyl-C(=O)R^{a}, -C₁-C₆-alkyl-C(=O)OR^{a}, -C₁-C₆-alkyl-C(=O)N(R^{a})₂, -C₁-C₆-alkyl-C(=S)N(R^{a})₂, -C₁-C₆-alkyl-NR^{a}C(=O)OR^{a}, -C₁-C₆-alkyl-NR^{a}C(=O)N(R^{a})₂,-C₁-C₆-alkyl-NR^{a}C(=S)N(R^{a})₂, -C₁-C₆-alkyl-NR^{a}C(=O)R^{a}, -C₁-C₆-alkyl-NR^{a}C(=S)R^{a}, -C₁-C₆-alkyl-OC(=O)N(R^{a})₂, -C₁-C₆-alkyl-NR^{a}S(=O)₂R^{a}, -C₁-C₆-alkyl-S(=O)₂R^{a}, -C₁-C₆-alkyl-S(=O)₂N(R^{a})₂, -C₁-C₆-alkyl-P(=O)(OR^{a})₂ substituent is itself optionally substituted, one or more times, in the same way or differently, with R5, wherein R^{a} and R5 are as defined in any one of claims 1 to 5 ; and E is a -C(O)H group ;
to provide a compound of general formula I : 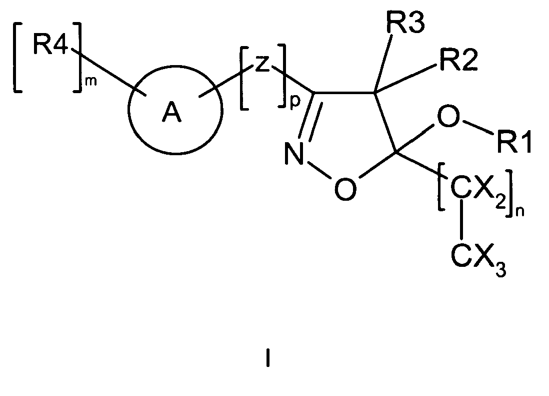 in which formula I, the definitions of A, R1, R2, R3, R4, X, Z, m, n, and p, are given in any one of claims 1 to 5.

8. A method of preparing a compound of general formula (I) according to any one of claims 1 to 5, said method comprising the step of allowing an intermediate ketone compound of general formula 1 b : 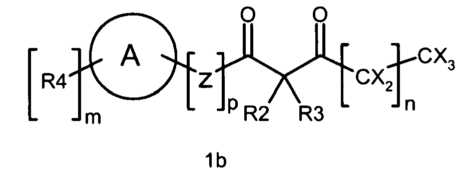 to react with hydroxylamine to provide a compound of general formula I : 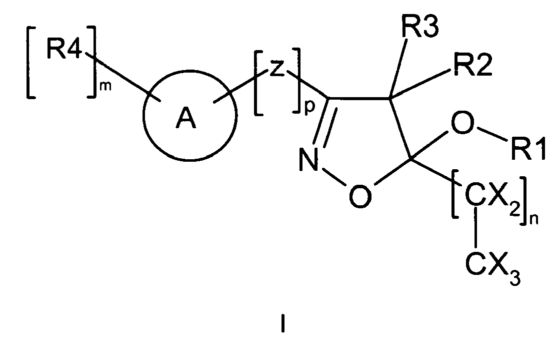 in which formulae 1 b and I, the definitions of A, R1, R2, R3, R4, X, Z, m, n, and p, are given in any one of claims 1 to 5.

9. A method of preparing a compound of general formula (I) according to any one of claims 1 to 5, said method comprising the step of allowing an intermediate oxime compound of general formula 2a : 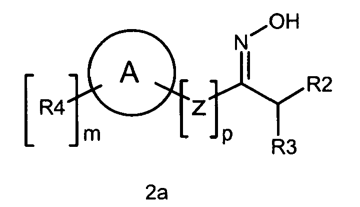 to react, in the presence of a base, such as n-butyllithium, with a compound of formula 2c : 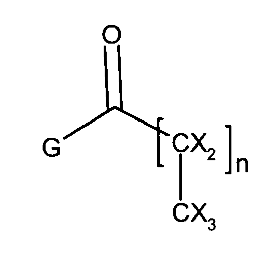 to provide a compound of general formula I : 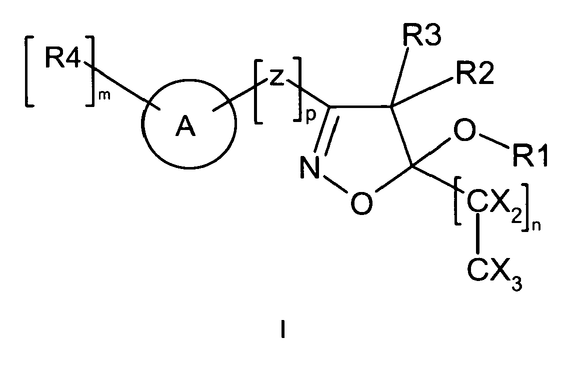 in which formulae 2a, 2c and I, the definitions of A, R1, R2, R3, R4, X, Z, m, n, and p, are given in any one of claims 1 to 5, and in which formula 2c, G is a leaving group.

10. A method of preparing a compound of general formula (I) according to any one of claims 1 to 5, said method comprising the step of allowing an intermediate ketone compound of general formula 3b : 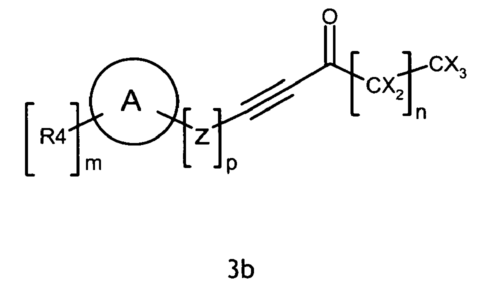 to react with hydroxylamine to provide a compound of general formula I : 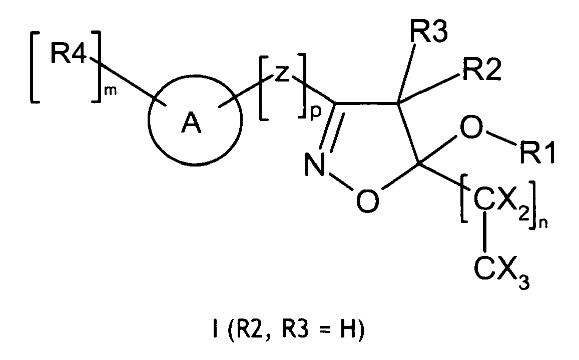 in which formulae 2a, 2c and I, the definitions of A, R1, R2, R3, R4, X, Z, m, n, and p, are given in any one of claims 1 to 5.

11. A method of preparing a compound of general formula (I) according to any one of claims 1 to 5, said method comprising the step of allowing an intermediate isoxazoline compound of general formula I : 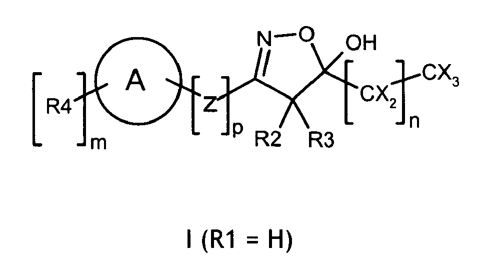 to react with an electophile of formula 4a :
E-R1 4a
to provide a compound of formula I : 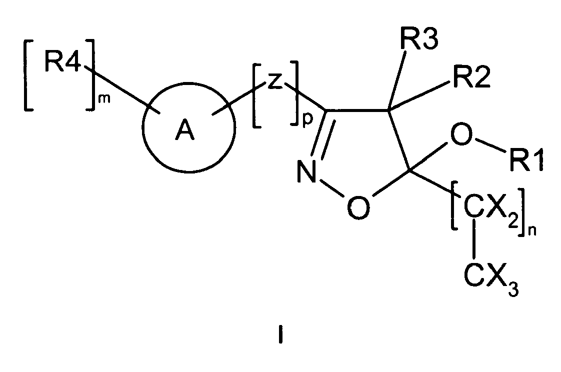 in which formula 4a and I, the definitions of A, R1, R2, R3, R4, X, Z, m, n, and p, are given in any one of claims 1 to 5, and in which formula 4a, E is a leaving group.

12. A pharmaceutical composition which comprises a compound of general formula (I) according to any one of claims 1 to 5, or a pharmaceutically acceptable salt or an in *vivo* hydrolysable ester thereof, and a pharmaceutically-acceptable diluent or carrier.

13. Use of a compound of any one of claims 1 to 5 for manufacturing a pharmaceutical composition for the treatment of diseases in which inhibition of histone deacetylase can prevent, inhibit or ameliorate the pathology and/or symptomatology of the disease.

14. Use according to claim 13, wherein said disease is a disease caused by increased cell proliferation.

15. Use according to claim 13, wherein said diseases are cancer, psoriasis, fibroproliferative disorders, smooth muscle cell proliferation disorders, inflammatory diseases and conditions treatable by immune modulation, neurodegenerative disorders, diseases involving angiogenesis, fungal and parasitic infections and haematopoietic disorders.

16. Use according to claim 13, wherein said diseases are liver fibrosis, arteriosclerosis, restenosis, rheumatoid arthritis, autoimmune diabetes, lupus, allergies, Huntington's disease, retinal diseases, protozoal infections, anaemia, sickle cell anaemia and thalassemia.

17. Use of claim 16, wherein said protozoal infection is malaria, toxoplasmosis or coccidiosis.

18. Use of claim 16, wherein said retinal disease is diabetic retinopathy, age-realted macular degeneration, interstitial keratitis or rubeotic glaucoma.

19. Use according to claim 13, wherein said disease is congestive heart failure due to cardiomyocyte hypertrophy.

20. A method of treating a disease in which inhibition of histone deacetylase can prevent, inhibit or ameliorate the pathology and/or symptomatology of the disease by administering an effective amount of a compound of general formula (I) according to any one of claims 1 to 5.

21. The method according to claim 20, wherein said disease is a disease caused by increased cell proliferation.

22. The method according to claim 20, wherein said diseases are cancer, psoriasis, fibroproliferative disorders, smooth muscle cell proliferation disorders, inflammatory diseases and conditions treatable by immune modulation, neurodegenerative disorders, diseases involving angiogenesis, fungal and parasitic infections and haematopoietic disorders.

23. The method according to claim 20, wherein said diseases are liver fibrosis, arteriosclerosis, restenosis, rheumatoid arthritis, autoimmune diabetes, lupus, allergies, Huntington's disease, retinal diseases, protozoal infections, anaemia, sickle cell anaemia and thalassemia.

24. The method according to claim 23, wherein said protozoal infection is malaria, toxoplasmosis or coccidiosis.

25. The method according to claim 23, wherein said retinal disease is diabetic retinopathy, age-realted macular degeneration, interstitial keratitis or rubeotic glaucoma.

26. The method according to claim 20, wherein said disease is congestive heart failure due to cardiomyocyte hypertrophy.
